# EUROPÄISCHE PATENTSCHRIFT

(11) **EP 3 522 835 B1**
(45) Veröffentlichungstag und Bekanntmachung des Hinweises auf die Patenterteilung: **16.03.2022**
(21) Anmeldenummer: 17787359.3
(22) Anmeldetag: 06.10.2017
(51) Int. Cl.: A61F 2/64

(54) **GELENKEINRICHTUNG, HYDRAULIKEINHEIT UND VERFAHREN ZUR STEUERUNG EINER GELENKEINRICHTUNG**
ARTICULATION DEVICE, HYDRAULIC DEVICE AND PROCEDURE FOR OPERATING AN ARTICULATION DEVICE
DISPOSITIF ARTICULE, DISPOSITIF HYDRAULIQUE ET PROCEDURE POUR OPERER UN DISPOSITIF ARTICULE

(30) Priorität: 06.10.2016 DE 102016118999
(43) Veröffentlichungstag der Anmeldung: 14.08.2019
(73) Patentinhaber: Otto Bock Healthcare Products GmbH, 1070 Wien (AT)
(72) Erfinder: SEYR, Martin, 1040 Wien (AT); SIMA, Harald, 3130 Herzogenburg (AT); AUBERGER, Roland, 1140 Wien (AT)
(74) Vertreter: Gramm, Lins & Partner Patent- und Rechtsanwälte PartGmbB
(86) Internationale Anmeldenummer: PCT/EP2017/075573
(87) Internationale Veröffentlichungsnummer: WO 2018/065615

(56) Entgegenhaltungen:
- DE-U1- 9 405 545
- US-A- 3 605 960
- US-A- 5 948 021
- US-B2- 6 997 959

## Beschreibung

Die Erfindung betrifft eine Gelenkeinrichtung mit einem Oberteil und einem Unterteil, die um eine Schwenkachse verschwenkbar aneinander gelagert sind, mit einer an dem Oberteil und dem Unterteil befestigten Hydraulikeinheit, mit einem Gehäuse, in dem ein Zylinder angeordnet ist, in dem ein mit einer Kolbenstange verbundener Arbeitskolben verlagerbar gelagert ist und den Zylinder in zwei Kammern unterteilt, wie in Ansprüchen 1-12 definiert.

Die Erfindung betrifft ebenfalls eine solche Hydraulikeinheit, wie in Anspruch 13 definiert und ein Verfahren zum Einstellen und Betreiben einer solchen Gelenkeinrichtung, wie in Ansprüchen 14-22 definiert, die insbesondere in orthetischen oder prothetischen Vorrichtungen eingesetzt werden können. Unter orthetischen oder prothetischen Vorrichtungen werden insbesondere Orthesen, Prothesen und Exoskelette verstanden. Die Hydraulikeinheit kann auch in der Robotik eingesetzt werden.

Bei orthetischen oder prothetischen Vorrichtungen werden häufig zwei Komponenten relativ zueinander verlagert, beispielsweise werden ein Oberteil und ein Unterteil um eine Gelenkachse bei einem Orthesengelenk oder Prothesengelenk relativ zueinander verschwenkt, ebenso können längsverschiebliche Relativbewegungen zwischen zwei orthetischen oder prothetischen Komponenten auftreten. Häufig ist es notwendig, die Relativbewegung zu dämpfen. Zu diesem Zweck werden hydraulische und/oder pneumatische Dämpfer eingesetzt, die eine Extension oder Flexion eines Drehgelenkes oder einer Verschiebebewegung in die eine oder andere Richtung dämpfen. Die Dämpfer können einstellbar ausgestaltet sein, um auf der Grundlage von Sensordaten oder festgelegten Steuerkurven über den Verlauf einer Bewegung veränderte Widerstände gegen die Relativbewegung bereitzustellen. Hierzu sind in Strömungskanälen Ventile oder Drosseln vorgesehen, die gegebenenfalls einstellbar sind.

Um eine Bewegung der Komponenten der orthetischen oder prothetischen Vorrichtung zu unterstützen, sind Aktuatoren vorgesehen, die beispielsweise als Elektromotoren ausgebildet sein können. Ebenfalls können hydraulische oder pneumatische Antriebe vorgesehen sein. Über Energiespeicher ist es möglich, kinetische Energie zu speichern, so dass im weiteren Verlauf des Bewegungszyklusses oder zu einem anderen Zeitpunkt diese gespeicherte Energie, die beispielsweise durch Abbremsen einer Flexionsbewegung erhalten wurde, wieder dem System zugeführt werden kann. Über die Rückführung der Energie wird die orthetische oder prothetische Vorrichtung angetrieben, so dass ein Aktuator vorliegt. Über einen Aktuator wird Energie einer Bewegung unterstützend zugeführt.

Aus der DE 10 2012 013 141 A1 ist eine orthetische oder prothetische Gelenkeinrichtung mit einem Oberteil und einem schwenkbar daran angeordneten Unterteil mit zumindest einer Hydraulikeinheit zwischen dem Oberteil und dem Unterteil bekannt, die einen in einem Gehäuse mit einer Extensionskammer und Flexionskammer beweglichen Kolben aufweist, der mit dem Oberteil oder dem Unterteil gekoppelt ist. Über eine Druckbereitstellungseinrichtung kann der Kolben mit einem Druck beaufschlagt werden, wobei die Druckbereitstellungseinrichtung zumindest einen Druckspeicher aufweist, der über eine Umschalteinrichtung wahlweise mit der Extensionskammer oder mit der Flexionskammer koppelbar ist. Der Druckspeicher kann mit einer Pumpe gekoppelt sein, um über die Pumpe den Druckspeicher aufzufüllen. Nachteilig an einer solchen Konstruktion ist der vergleichsweise hohe Raumbedarf durch den externen Druckspeicher.

Die US 5,948,021 A betrifft ein Prothesenkniegelenk mit einem Oberteil und einem Unterteil, die über ein Mehrlenkersystem miteinander schwenkbar gekoppelt sind. Eine hydraulische Steuereinheit ist zwischen dem Oberteil und dem Unterteil vorgesehen, um eine Flexionsdämpfung und Extensionsdämpfung bereitzustellen. Die hydraulische Steuereinheit weist ein Zylinderpaar auf, bei dem die Zylinder exzentrisch zueinander ausgerichtet sind. Ein Kolben ist an einer Kolbenstange gelagert und erstreckt sich durch ein Ölreservoir innerhalb eines der beiden Zylinder. Eine Feder oder ein Gasvolumen ist auf der dem Ölreservoir abgewandten Seite des Kolbens angeordnet.

Die US 6,997,959 B2 betrifft ein Prothesenkniegelenk mit einem Oberteil und einem Unterteil, die über ein Mehrlenkersystem gelenkig aneinander gelagert sind. Eine Kolbenstange ist an dem Oberteil befestigt und bewegt bei einer Flexion oder Extension einen Kolben in einem mit Hydraulikfluid gefüllten Zylinder. Über Drosseln werden Widerstände eingestellt. Zusätzlich ist an der Kolbenstange ein Pneumatikkolben angeordnet. Die Kolbenstange stützt sich gegenüber einer Druckfeder ab.

Die US 3,605,960 A betrifft einen hydraulischen Stoßdämpfer mit einem Gehäuse, in dem ein Zylinder angeordnet ist, in dem ein Kolben an einer Kolbenstange geführt ist. Innerhalb des Zylinders ist eine Druckfeder angeordnet, die eine Rückstellung des Kolbens bewirkt.

Die DE 94 05 545 U1 betrifft eine Schwungphasensteuerungseinrichtung für ein künstliches Kniegelenk mit einem Zylinder, in dem ein Kolben an einer Kolbenstange längsverschieblich geführt ist. Der Kolben unterteilt den Zylinder in eine Extensionskammer und eine Flexionskammer, die bei der jeweiligen Bewegung das darin befindliche Volumen komprimieren. Die Extensionskammer weist einen Verbindungskanal zur Umgebung mit einem sperrenden Rückschlagventil auf und ist mit der Flexionskammer durch einen Drosselkanal verbunden. Die Flexionskammer weist eine Belüftungsbohrung in die Umgebung sowie eine Druckfeder auf.

Bei Prothesenfüßen oder Orthesen mit einem Fußteil, bei denen eine Hydraulikeinheit eingesetzt wird, um eine Gelenkeinrichtung zu dämpfen oder das Verhalten der Gelenkeinrichtung zu steuern, besteht das Problem, dass eine aufwendige Anpassung an unterschiedliche Absatzhöhen erfolgen muss. Eine Verschiebung der Nullpunktlage eine Federeinrichtung ist kompliziert. Darüber hinaus sind Schwingvorgänge um eine Ausgangslage ohne Verstellvorgänge nicht vorgesehen. Dissipative Vorgänge können nicht einfach mit Energie speichernden Vorgängen verbunden werden.

Aufgabe der vorliegenden Erfindung ist es daher, eine Gelenkeinrichtung, eine Hydraulikeinheit und ein Verfahren zum Einstellen und Betrieben einer Gelenkeinrichtung bereitzustellen, mit denen unterschiedliche Bewegungen und Dämpfungseigenschaften mit einem möglichst geringen Verstellaufwand erreicht werden können.

Erfindungsgemäß wird diese Aufgabe durch eine Gelenkeinrichtung mit den Merkmalen von Anspruch 1 sowie mit einer Hydraulikeinheit gemäß Anspruch 13 und einem Verfahren gemäß Anspruch 14 sowie mit den Merkmalen der jeweiligen Nebenansprüche gelöst. Vorteilhafte Ausgestaltungen und Weiterbildungen der Erfindung sind in den Unteransprüchen, der Beschreibung sowie den Figuren offenbart.

Die erfindungsgemäße Gelenkeinrichtung mit einem Oberteil und einem Unterteil, die um eine Schwenkachse verschwenkbar aneinander gelagert sind, mit einer an dem Oberteil und dem Unterteil befestigten Hydraulikeinheit, mit einem Gehäuse, in dem ein Zylinder angeordnet ist, in dem ein mit einer Kolbenstange verbundener Arbeitskolben verlagerbar gelagert ist und den Zylinder in zwei Kammern unterteilt sieht vor, dass der Arbeitskolben mit zumindest einer zug- und druckkraftübertragenden Federeinrichtung gekoppelt ist, die an zumindest einem Widerlager angreift, das innerhalb des Zylinders angeordnet ist. Die Kopplung der Federeinrichtung mit dem Arbeitskolben kann entweder unmittelbar mechanisch oder hydraulisch erfolgen. Eine hydraulische Kopplung liegt vor, wenn die Federeinrichtung über ein hydraulisches Medium, beispielsweise Hydrauliköl oder Wasser, auf den Arbeitskolben wirkt. Dazu ist die Federeinrichtung mit einem weiteren Hydraulikelement gekoppelt, beispielsweise einem Kolben. Über die verlagerbare Anordnung des Widerlagers oder der Widerlager und des Arbeitskolbens innerhalb einer Kammer besteht die Möglichkeit, die Position des Widerlagers oder der Widerlager zu verändert und damit die ihm zugeordnete Federeinrichtung vorzuspannen oder bei der Entspannung der Federeinrichtung Kräfte auf den Arbeitskolben zu übertragen. Ebenso kann dadurch auch die Position des Arbeitskolbens innerhalb der Kammer verlagert werden. Insbesondere ist es möglich, bei einem einstellbaren Nulldurchgang, um den herum die Gelenkeinrichtung verschwenkt, ohne weitere Verstellung der Widerlager oder der Federeinrichtung ein gewünschtes oder vorab eingestelltes Federverhalten beizubehalten. Nach der Einstellung eines Nulldurchganges ist üblicherweise kein oder nur ein geringer Aufwand notwendig, um das weitere Verhalten der Gelenkeinrichtung zu steuern. Über die beidseitig wirkende Federeinrichtung, die in Zugkraftrichtung und in Druckkraftrichtung wirkt, ist es möglich, die Gelenkeinrichtung um den Nullpunkt oder den Nulldurchgang, bei dem sich die Zugkräfte und Druckkräfte in einem Gleichgewicht befinden, herum auszulenken, so dass in beiden Richtungen der Verschwenkung um den Nulldurchgang eine Energiespeichermöglichkeit von der Federeinrichtung bereitgestellt wird.

In einer Weiterbildung der Erfindung ist es vorgesehen, dass das Widerlager als ein verschieblich gelagertes Trennelement oder Kolben in der Kammer gelagert ist, so dass das Trennelement oder der Kolben von der Kammer eine Teilkammer abteilt. Die von dem Arbeitskolben in dem Zylinder unterteilten beiden Kammern, die auch als Extensionskammer und Flexionskammer bezeichnet werden können, werden durch das Trennelement oder einen weiteren Kolben, der als Widerlager für die Federeinrichtung wirkt, in zwei Unterkammern unterteilt, von denen eine als Kammer und die andere als Teilkammer bezeichnet wird. Die dem Arbeitskolben zugewandte Kammer wird als Kammer und die auf der dem Kolben abgewandten Seite des Trennelementes ausgebildete Kammer wird als Teilkammer bezeichnet. Durch eine solche Ausgestaltung ist es möglich, die Federwirkung leicht zu sperren, abzuschalten oder auch zu verändern, indem eine hydraulische Verbindung von der Teilkammer in die Kammer oder in eine andere Kammer oder Teilkammer gesperrt oder gedrosselt wird. Eine solche Sperrung oder Veränderung der Federeigenschaften oder der Dämpfungseigenschaften lässt sich besonders leicht über ein Stellventil erreichen, das in einem Kanal oder in einer Verbindungsleitung zwischen Kammer und Teilkammer oder zwischen Teilkammern angeordnet ist.

Die Kammern und Teilkammern sind bevorzugt hydraulisch miteinander über Kanäle gekoppelt, in denen einstellbare Ventile angeordnet sind. Über diese Ventile ist es möglich, die Bewegung des Arbeitskolbens zu sperren oder unter Last nachgebende Bewegungen des Arbeitskolbens zuzulassen, ohne dass die Federkennlinie sich wesentlich ändert. Darüber hinaus kann der Nullpunkt der jeweiligen Federeinrichtung einstellbar sein, also diejenige Lage, in der Zugkräfte und Drucckräfte im Gleichgewicht befindlich sind. Wenn alle Kammern und Teilkammern hydraulisch miteinander verbunden und über Ventile der jeweilige Zugang gedrosselt oder gesperrt oder geöffnet werden kann, sind eine Vielzahl von Kombinationen aus Federwirkungen oder Sperrungen der Gelenkeinrichtung möglich, ohne dass die Ventile bei der Änderung der Bewegungsrichtung und/oder Kraftrichtung umgestellt werden müssten. Die Position der Widerlager kann durch Volumenveränderungen vorgenommen werden, die einfach während der Bewegung durch Ventilverstellungen verwirklicht und fixiert werden können. Ebenso ist es möglich, eine Energiespeicherung in einer bestimmten Bewegungsphase oder Verlagerungsposition der Gelenkeinrichtung vorzunehmen, ein Hydraulikvolumen mit einer belasteten Federeinrichtung zu sperren und zu einem späteren Zeitpunkt freizugeben.

Das Widerlager kann die Kammern nach außen begrenzen, wobei eine unverschiebliche Sperrwand die Teilkammern von den Kammern abteilt. Auf der dem Arbeitskolben oder der Kammer abgewandten Seite des Widerlagers muss kein Hydraulikfluid vorhanden sein, insbesondere wird für eine einfache Konstruktion die Federeinrichtung außerhalb der Kammer angeordnet werden. Die Kammern und Teilkammern können über in der Sperrwand angeordnete, verstellbare Ventile miteinander verbunden sein, um die Nullpunktlage sowie das Schwingverhalten oder Bewegungsverhalten der Gelenkeinrichtung bei Belastung zu verändern oder um das gewünschte Verhalten einstellen zu können.

Die Federeinrichtung kann als eine Zug-Druckfeder oder als eine Kombination aus zwei gegenläufig wirkenden Zug- und Druckfedern oder als eine Kombination aus zwei gegenläufig wirkenden Zug- oder Druckfedern ausgebildet sein. Der Einsatz einer kombinierten Zug-Druckfeder hat den Vorteil einer Verringerung der Teilanzahl, der Verringerung des Gewichtes sowie einem verringerten Montageaufwand, wohingegen die Trennung der Federeinrichtung in Zugfeder und Druckfeder oder entgegengesetzt wirkende Zug- oder Druckfedern eine größere Vielfalt an Einstellmöglichkeiten eröffnet, da die jeweiligen Federn auf die jeweilige Belastung ausgerichtet und an die jeweiligen Beanspruchungen angepasst werden können.

Eine Weiterbildung der Erfindung sieht vor, dass die Federeinrichtung innerhalb einer Kammer, insbesondere einer Teilkammer angeordnet ist, wodurch sich ein minimaler Bauraum und eine Integration sämtlicher hydraulischer Komponenten und Federkomponenten innerhalb der Hydraulikeinheit realisieren lässt, so dass eine kompakte Bauweise sowohl der Hydraulikeinheit als auch der Gelenkeinrichtung möglich ist. Die Hydraulikeinheit stellt dann ein geschlossenes System dar, bei dem über Ventile die Strömungswiderstände des Hydraulikfluids zwischen den Kammern und Teilkammern eingestellt werden können.

Die Zug- oder Druckfeder kann in unterschiedlichen Teilkammern oder auf einer Seite des Arbeitskolbens in einer Kammer und Teilkammer angeordnet sein, um über verschiedene Federn und verschiedene Kammern unterschiedliche Federeigenschaften und Energiespeichereigenschaften bereitstellen zu können.

Eine Weiterbildung der Erfindung sieht vor, dass die Federeinrichtung das Oberteil relativ zu dem Unterteil in einer einstellbaren Ausgangsstellung hält. Dies ist beispielsweise bei einer Ausgestaltung der Gelenkeinrichtung als ein Knöchelgelenk vorteilhaft, um eine Anpassung auf unterschiedliche Absatzhöhen oder auf eine Anpassung an abweichende Untergrundneigungen vornehmen zu können. Bei einer Ausgestaltung der Gelenkeinrichtung als Knöchelgelenk, beispielsweise in einem Prothesenfuß oder einem Orthesenfuß, beinhaltet die normale Gangbewegung und Abrollbewegung eine Aufladung der Feder während der Plantarflexion nach einem Fersenkontakt. Anschließend entlädt sich die Federeinrichtung während einer Dorsalflexion in der ersten Hälfte der Überrollbewegung. Nach Erreichen einer Ausgangsstellung lädt sich bei einem weiteren Überrollen bei zunehmender Dorsalflexion die Federeinheit erneut auf und entlädt sich anschließend in der Entlastungsphase bis zur Zehenablösung ein letztes Mal. Dabei wird der Fuß während einer Plantarflexion zurück in die Ausgangsstellung geführt. Die in Zug- und Druck-richtung wirkende Federeinrichtung hält somit das Oberteil relativ zu dem Unterteil oder den Fuß relativ zu dem Unterschenkelteil in einer Ausgangsstellung, um die herum der Fuß verschwenkt. Die Verschwenkung erfolgt gegen die Rückstellkraft der Federeinrichtung sowohl in Plantarflexionsrichtung als auch in Dorsalflexionsrichtung. Durch die verlagerbare Ausgestaltung des Widerlagers/der Widerlager und/oder des Arbeitskolbens ist es möglich, eine Anpassung an geändertes Schuhwerk, insbesondere bei einer Veränderung der Absatzhöhe, oder bei der Ausführung besonderer Bewegungsmuster vorzunehmen, ohne dass weitere Veränderungen in der Steuerung vorgenommen werden müssten. Die Federeigenschaften verändern sich im Wesentlichen nicht durch die Veränderung des Nulldurchgangs oder durch die Veränderung der Ausgangsstellung. Grundsätzlich ist es jedoch auch möglich, während des Gehens oder während der Bewegung der Gelenkeinrichtung die Position des Widerlagers oder der Widerlager zu verstellen, so dass die Federbewegung der Federeinrichtung durch ein passives Nachgeben in Plantarflexionsrichtung und/oder Dorsalflexionsrichtung überlagert werden kann. Der Stellaufwand für die Ventile zum Verstellen der Position des Widerlagers oder einer anderen Verstelleinrichtung zum Verstellen des Widerlagers ist vergleichsweise gering, die Bewegung, insbesondere wenn keine Überlagerung durch passives Nachgeben erfolgt, ist üblicherweise elastisch, also energiespeichernd und energieabgebend während der Plantarflexion und der Dorsalflexion, je nach Bewegungsphase und im Wesentlichen nicht dissipierend, was zu einem erleichterten Gehen führt. Darüber hinaus ist es möglich, neben einer Absatzhöhenanpassung und einer Geländeanpassung die Federeinrichtung zu blockieren, beispielsweise für das Stehen, so dass eine verbesserte Standstabilität gewährleistet ist.

Bei einer Ausgestaltung der Gelenkeinrichtung als ein Prothesenkniegelenk oder Orthesenkniegelenk kann die Ausgangsstellung ebenfalls frei gewählt werden, beispielsweise geringfügig vor Erreichen einer Maximalstreckung mit einem Kniegelenkswinkel von 180°. Das aus der Schwungphase kommende Kniegelenk durchläuft dann vor Erreichen des maximalen Kniewinkels die Ausgangsstellung oder den Nulldurchgang, beispielsweise in einem Bereich von 3 Grad bis 8 Grad vor Erreichen eines Kniewinkels von 180 Grad, um dann in der Endphase der Schwungphase die Federeinrichtung zu komprimieren. Die Federsteifigkeit und ebenso die Ausgangsstellung werden so gewählt, dass aufgrund der Massenträgheit des Unterschenkelteils und des gegebenenfalls daran befindlichen Fußteils eine maximale Extension auch gegen die Federeinrichtung erreicht wird. Bei dem Aufsetzen oder Heel Strike befindet sich das Kniegelenk in einer gestreckten, jedoch aufgrund der vorgespannten Federeinrichtung in Richtung auf eine Flexion vorgespannten Stellung, so dass sich eine Unterstützung der Standphasenflexion direkt nach dem Fersenkontakt ergibt durch die sich entspannende Federeinrichtung.

Ein solches Verhalten kommt dem physiologischen Gangmuster sehr nahe. Anschließend wird die Federeinrichtung während der zunehmenden Standphasenbeugung in Flexionsrichtung aufgeladen. Nach Erreichen der maximalen Standphasenflexion wird die Feder in Standphasenextension wieder entladen, um sich während des Roll Over über die Ausgangsstellung hinaus wieder in die Standphasenextension mit einer maximalen Streckung aufzuladen. Die in der terminalen Standphase erneut aufgeladene Federeinrichtung wird dann bei der Schwungphaseneinleitung wieder abgegeben und die Schwungphasenflexion unterstützen. Dieses Verhalten kann ohne oder nur mit minimalen Ventileinstellvorgängen oder Verstelleinrichtungen von Widerlagern vor sich gehen. Während des Stehens kann durch Verstellung des Widerlagers/der Widerlager und/oder des Arbeitskolbens und gegebenenfalls Entkopplung der Federeinrichtung eine maximale Extension und damit eine stabile Gelenkeinrichtung auf einfache Art und Weise erreicht werden, so dass bei einer Maximalextension keinerlei flektierende Momente auf die Gelenkeinrichtung wirken.

Um unterschiedliche Bewegungsmuster zu erkennen, kann ein Sensor in der Gelenkeinrichtung angeordnet oder der Gelenkeinrichtung zugeordnet werden, beispielsweise ein Inertialwinkelsensor, mehrere Winkelsensoren, die Winkelstellungen zueinander von Oberteil und Unterteil oder auch anderen Orthesen und Prothesenkomponenten erfassen, oder andere Lagesensoren und/oder Geschwindigkeitssensoren und/oder Beschleunigungssensoren, um die jeweiligen Gangphasen und Bewegungsmuster zu erfassen.

Die Gelenkeinrichtung ermöglicht insbesondere ein Schwingen um einen einmal eingestellten Nullpunkt oder um einen variablen, einstellbaren Nullpunkt, ohne dass eine weitere Verstellung von Ventilen während des weiteren Bewegungsablaufes erfolgt. Die übrigen Feder- und Dämpferverhalten werden durch die Federeinrichtung und die Strömungsbedingungen innerhalb der Hydraulikeinheit festgelegt. Die Federeinrichtung ist abschaltbar, indem die Hydraulikeinheit durch Schließen der Ventile gesperrt wird. Durch ein Verstellen des Widerlagers während des Gehens können dissipative Komponenten durch die Hydraulikeinheit verwirklicht werden. Besonders einfach kann die Gelenkeinrichtung betrieben werden, wenn zwei Widerlager in der Hydraulikeinrichtung vorhanden sind, die auf einander gegenüberliegenden Seiten des Arbeitskolbens angeordnet sind, so dass sowohl in Flexionsrichtung als auch in Extensionsrichtung unterschiedliche Positionen des Widerlagers oder der Widerlager eingestellt werden können.

Die Erfindung betrifft ebenfalls eine Hydraulikeinheit, wie sie oben beschrieben worden ist, auch ohne dass sie in einer Gelenkeinrichtung eingebaut ist.

Das Verfahren zum Einstellen und Betreiben einer wie oben beschriebenen Gelenkeinrichtung mit einem Oberteil und einem Unterteil, die um eine Schwenkachse verschwenkbar aneinander gelagert sind, mit einer an dem Oberteil und dem Unterteil befestigten Hydraulikeinheit, mit einem Gehäuse, in dem ein Zylinder angeordnet ist, in dem ein mit einer Kolbenstange verbundener Arbeitskolben verlagerbar gelagert ist und den Zylinder in zwei Kammern unterteilt, sieht vor, dass der Arbeitskolben mit zumindest einer zug- und druckkraftübertragenden Federeinrichtung gekoppelt wird, die an zumindest einem verlagerbar, vorzugsweise in einer Kammer verlagerbar gelagerten Widerlager angreift, wobei die Einstellung einer Nullpunktlage des Arbeitskolbens oder eine Verschwenkbewegung des Oberteils relativ zu einem Unterteil durch Öffnen oder Verstellen von Ventilen in Kanälen durchgeführt wird. Das zumindest eine innerhalb der Kammer angeordnete Widerlager überträgt die hydraulischen Kräfte auf die Federeinrichtung und zurück auf den Arbeitskolben und kann zur Einstellung einer Nullpunktlage oder während der Verschwenkbewegung verstellt werden. Durch die Verstellung der Nullpunktlage der Hydraulikeinheit ist es möglich, eine Ausgangsstellung oder eine Nullpunktlage einzustellen, in der die jeweilige Federeinrichtung nicht wirksam in Eingriff gebracht wird. Die Gelenkeinrichtung kann so eingestellt werden, dass eine Anpassung an unterschiedliche konstruktive Ausgestaltungen, beispielsweise an unterschiedliche Absatzhöhen oder an zu erreichende Gelenkwinkel, erreicht wird. Wird das Widerlager oder werden die Widerlager während der Verschwenkbewegung bewegt oder verstellt, wird eine dissipative oder Energie speichernde Komponente hinzugefügt, wobei der Verstellaufwand aufgrund der Kombination der Verschwenkbewegung mit einer Federeinrichtung vergleichsweise gering gehalten werden kann. Durch die Federeinrichtung ist es möglich, einen freien oder nahezu freien Kraftfluss beim Einsatz der Gelenkeinrichtung in einer orthopädietechnischen Einrichtung wie einer Prothese oder Orthese zu gewährleisten, so dass eine Verschwenkbewegung um die Ausgangsstellung oder Nullpunktlage ohne eine weitere Verstellung der Widerlager oder aber durch Zu- und Abschalten von Drosseln erreicht werden kann.

Das Widerlager beziehungsweise die Position des Widerlagers wird bevorzugt hydraulisch verstellt. Dazu ist es bevorzugt vorgesehen, dass das Widerlager innerhalb einer der von dem Arbeitskolben unterteilten Kammern angeordnet ist und eine Teilkammer abteilt, die hydraulisch mit der jeweiligen Kammer verbunden ist. Über verstellbare Ventile kann die Position des Widerlagers bei Aufbringen einer entsprechenden Kraft auf eine Gelenkeinrichtung eingestellt und fixiert werden. Alternativ wird das Widerlager motorisch verstellt, beispielsweise elektromotorisch über ein Getriebe und eine Verstelleinrichtung, beispielsweise Gewinde.

Das Widerlager kann die Kammer nach außen begrenzen, wobei eine vorzugsweise unverschiebliche, zumindest fixierbare Sperrwand die Teilkammern von den Kammern abteilt und die Kammern über Leitungen miteinander hydraulisch gekoppelt werden. Die jeweilige Kammer und die Teilkammer werden über einstellbare Ventile verbunden, wobei die Ventile zur Sperrung der Gelenkeinrichtung geschlossen werden. Zur Freigabe der Gelenkeinrichtung werden die hierzu notwendigen Ventile geöffnet.

Das Widerlager teilt von der Kammer bevorzugt eine Teilkammer ab, vorteilhafterweise sind auf beiden Seiten des Arbeitskolbens, also in beiden Kammern, jeweils ein Widerlager angeordnet, so dass zwei Teilkammern ausgebildet werden, die auf einander gegenüberliegenden Seiten des Arbeitskolbens angeordnet sind. Die Kammern und die Teilkammern oder die Teilkammer, wenn nur eine Teilkammer vorhanden ist, sind über Leitungen und Ventile hydraulisch miteinander gekoppelt, wobei die Ventile zur Sperrung der Gelenkeinrichtung geschlossen werden. Beispielsweise während des Stehens ist es möglich und vorteilhaft, wenn die Gelenkeinrichtung blockiert wird, um eine maximale Sicherheit für den Nutzer der Gelenkeinrichtung in einer orthopädietechnischen Einrichtung wie beispielsweise eine Orthese oder Prothese bereitzustellen. Wenn alle Ventile geschlossen sind, kann keine Verlagerung der Gelenkeinrichtung um die Schwenkachse erfolgen. Alternativ ist es vorgesehen, dass zur Freigabe der Gelenkeinrichtung die den Kammern zugeordneten Ventile geöffnet werden, was beispielsweise während der Schwungphase bei einem Einsatz der Gelenkeinrichtung als ein Kniegelenk möglich ist.

Zur Dämpfung der Verschwenkbewegung können die Ventile nur teilweise geöffnet werden, so dass eine dissipative Komponente aufgrund eines erhöhten Strömungswiderstandes innerhalb der Kanäle oder Verbindungen zwischen den einzelnen Kammern oder Teilkammern eingestellt wird.

Zum Schwingen um einen einstellbaren Nullpunkt kann die hydraulische Verbindung zwischen zwei auf gegenüberliegenden Seiten des Arbeitskolbens liegende Teilkammern geschlossen und die Ventile zwischen den Kammern auf den gegenüberliegenden Seiten des Arbeitskolbens zumindest teilweise geöffnet werden, was beispielsweise bei einem Knöchelgelenk für das Gehen in der Ebene ein vorteilhafter Zustand wäre. Die Verbindung zwischen den Kammern wird dabei bevorzugt geschlossen.

Eine Weiterbildung des Verfahrens sieht vor, dass das Widerlager die Kammer nach außen begrenzt und eine unverschiebliche Sperrwand die Teilkammern von den Kammern abteilt und die Kammern über Leitungen miteinander hydraulisch gekoppelt sind sowie jeweils die Kammer und die Teilkammer über in der Sperrwand angeordnete einstellbare Ventile verbunden sind und zum Schwingen um einen einstellbaren Nullpunkt die hydraulische Verbindung zwischen zwei auf gegenüberliegenden Seiten des Arbeitskolbens liegende Kammern geschlossen und die Ventile zwischen jeweils den gegenüber des Arbeitskolben liegenden Kammern und den Teilkammer zumindest teilweise geöffnet werden.

Der Gelenkeinrichtung kann zumindest ein Sensor zugeordnet sein, mit einer Steuerungseinrichtung gekoppelt ist und die eine Verstellung des Widerlagers auf der Grundlage von Sensorwerten der Sensoren, die der Steuerungseinrichtung zugeordnet oder mit der Steuerungseinrichtung gekoppelt sind, ausführt. Die Sensoren sind insbesondere Lagesensoren, Winkelsensoren oder Neigungssensoren, die an dem Oberteil und/oder Unterteil angeordnet sind und Aussagen über Winkelstellungen oder Raumlagen des Oberteils und/oder des Unterteils erfassen. Diese erfassten Sensorwerte werden der Steuerungseinrichtung übermittelt, die diese auswertet. Die Steuereinrichtung kann beispielsweise ein Mikroprozessor oder Computer sein, der der Gelenkeinrichtung zugeordnet oder an der Gelenkeinrichtung oder einer daran angeordneten Komponente befestigt ist. Über eine Verstelleinrichtung werden dann entweder Ventile zur hydraulischen Verstellung der Position des Widerlagers und/oder des Arbeitskolbens oder andere Einstelleinrichtungen für die Position des jeweiligen Widerlagers und/oder Arbeitskolbens verstellt, beispielsweise um eine Nullpunktlage einzustellen. Auf der Grundlage der während einer Bewegung von den Sensoren erfassten Parameter oder Kenngrößen kann dann auch während der Bewegung eine entsprechende Verstellung der Widerlager und/oder des Arbeitskolbens erfolgen, um eine Anpassung an bestimmte Bewegungsmuster, Umweltbedingungen, Umweltbedingungen wie Rampen oder Änderungen in der orthetischen oder prothetischen Vorrichtung auszugleichen oder darauf zu reagieren.

Nachfolgend wird die Erfindung anhand der beigefügten Figuren näher erläutert. Gleiche Bezugszeichen bezeichnen gleiche Komponenten. Es zeigen:
- Figur 1 -: eine schematische Darstellung einer Aktuator-Dämpfer-Einheit;
- Figur 2 -: eine Variante der Figur 1 im montierten Zustand;
- Figur 3 -: eine schematische Darstellung einer Variante mit zwei Zusatzkolben;
- Figuren 4 bis 9 -: Anwendungsbeispiele der Aktuator-Dämpfereinheit;
- Figur 10 -: eine schematische Darstellung eines Kolbens mit einstellbarem Energiespeicher;
- Figur 11 -: eine Variante der Figur 10;
- Figur 12 -: eine Schnittdarstellung eines mechanischen Druckregelventils;
- Figur 13 -: eine Einbausituation des Druckregelventils in schematischer Darstellung;
- Figuren 14 bis 19 -: schematische Schnittdarstellungen einer Aktuator-Dämpfereinheit mit drei Kolben in einem durch zwei Sperrwände in drei hydraulisch miteinander verbundenen Kammern unterteilten Zylindern;
- Figur 20 -: ein Anwendungsbeispiel der Hydraulikeinheit in einem Prothesenfuß;
- Figur 21 -: einen Winkelverlauf eines Prothesenfußes; sowie
- Figur 22 -: einen Kniewinkelverlauf während des Gehens.

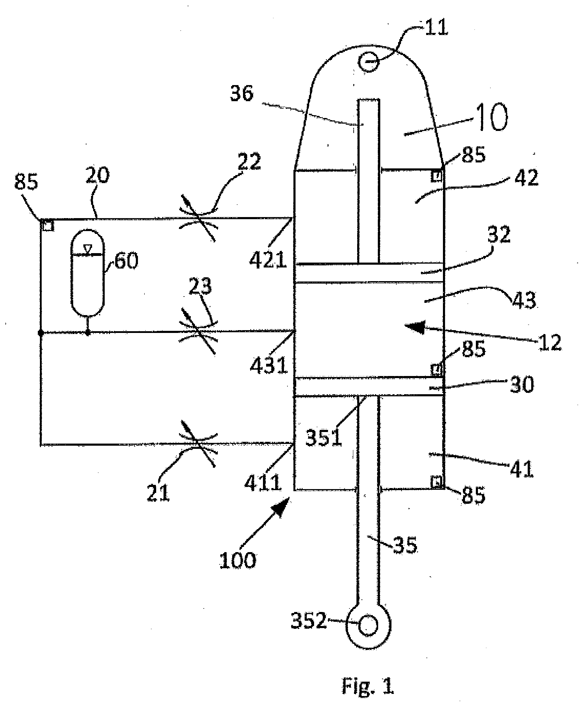

Figur 1 zeigt in einer schematischen Darstellung eine Aktuator-Dämpfer-Einheit 100 zum Einsatz in einer prothetischen oder orthetischen Vorrichtung, beispielsweise in einer Prothese oder Orthese. Die Aktuator-Dämpfer-Einheit, nachfolgend AD-Einheit, weist ein Gehäuse 10 auf, in dem ein Zylinder 12 ausgebildet ist. Im dargestellten Ausführungsbeispiel weist der Zylinder 12 einen kreisförmigen Querschnitt auf und nimmt in sich einen ersten Kolben 30 auf, der entlang der Zylinderwand verfahrbar gelagert ist. Die Form des Kolbens kann auch abweichend ausgebildet sein und kenn einen nicht kreisförmigen Querschnitt aufweisen. An dem ersten Kolben 30 ist eine Kolbenstange 35 angeordnet, deren erstes Ende 351 mit dem ersten Kolben 30 verbunden ist. Das zweite, dem ersten Ende 351 gegenüberliegende Ende 352 der Kolbenstange 35 ist an der prothetischen oder orthetischen Vorrichtung festlegbar. Das Gehäuse 10 weist eine Befestigungseinrichtung 11 auf, über die das Gehäuse 10 an einer anderen Prothesenkomponente oder Orthesenkomponente festlegbar ist. Werden die beiden Orthesen- oder Prothesenkomponenten relativ zueinander verlagert, wird der Kolben 30 innerhalb des Zylinders 12 bewegt, so dass sich eine Volumenveränderung einer ersten Fluidkammer 41 einstellt. Korrespondierend zu einer Volumenverringerung der ersten Fluidkammer 41 vergrößert sich das gegenüberliegende Volumen des Zylinders 12, das durch den ersten Kolben 30 unterteilt wird. In diesem zweiten Volumen ist ein zweiter Kolben 32 entlang der Längserstreckung des Zylinders 30 verschieblich angeordnet. Der zweite Kolben 32 unterteilt die zweite Fluidkammer 42, so dass sich zwischen den beiden Kolben 30, 32 eine dritte, volumenveränderbare Fluidkammer 43 ausbildet. Innerhalb jeder Fluidkammer 41, 42, 43 kann ein Sensor 85 in Gestalt eines Drucksensors angeordnet sein, um den in der jeweiligen Fluidkammer 41, 42, 43 vorherrschenden Druck erfassen zu können. In einer anderen Ausführungsform können andere Sensoren vorgesehen sein. Die Sensordaten der Sensoren 85 werden an eine Steuereinrichtung übermittelt, die später näher erläutert werden wird.

An dem zweiten Kolben 32 ist eine weitere Stange 36 angeordnet, die aus dem Gehäuse 10 herausführt. Weist die zweite Stange 36 den gleichen Querschnitt wie die Kolbenstange 35 auf, so muss bei einer reinen Verlagerung der beiden Kolben 30, 32 ohne Volumenveränderung der mittleren Fluidkammer 43 kein Ausgleichsvolumen für das transportierte Fluid bereitgestellt werden. Ein solches Ausgleichsvolumen ist notwendig, wenn nur eine Kolbenstange 35 vorhanden ist und das Fluid, das bevorzugt und im dargestellten Ausführungsbeispiel als Hydraulikfluid ausgebildet ist, im Wesentlichen inkompressibel ist. Verzichtet man auf die weitere Stange 36, muss das von der Kolbenstange verdrängte Volumen ausgeglichen werden, z.B. über ein Ausgleichsvolumen.

Jede der Fluidkammern 41, 42, 43 ist mit einer Zugangsöffnung 411, 421, 431 versehen, über die das Hydraulikfluid aus der jeweiligen Fluidkammer 41, 42, 43 aus-und wieder hineinströmen kann. Die Zugangsöffnungen 411, 421, 431 sind über Fluidleitungen 20 miteinander verbunden. Vor jeder Zugangsöffnung 411, 421, 431 ist ein Schalt- oder Stellventil 21, 22, 23 in der Fluidleitung 20 angeordnet, um den Strömungsquerschnitt der Fluidleitung 20 und damit auch den hydraulischen Widerstand einstellen zu können.

Innerhalb der weiteren, volumenveränderbaren Fluidkammer 43 kann ein kompressibles Medium oder eine Feder angeordnet sein, so dass bei einem geschlossenen Ventil 22 und einem zumindest teilweise geöffneten Ventil 23 das Volumen der dritten Fluidkammer 43 verringert wird. Dadurch wird das kompressible Medium komprimiert und Energie gespeichert. Durch die Volumenveränderung innerhalb der Fluidkammer 43 ist es notwendig, dass ein Ausgleichsvolumen 60 der AD-Einheit zugeordnet ist, so dass ein Volumenausgleich, z.B. aufgrund einer Leckage, einer einfahrenden Kolbenstange oder Temperaturschwankungen, stattfinden kann. Über einen optionalen Drucksensor 85 kann der Druck in dem Ausgleichsvolumen 60 gemessen werden, dies gibt Aufschluss über die Verringerung des Vordruckes aufgrund von Fluidverlusten. Wird das Ventil 23 geschlossen, verhält sich die dritte Fluidkammer 43 quasi starr, so dass eine normale Dämpferhydraulik bereitgestellt werden kann, beispielsweise mit einer Flexionskammer 41 und einer Extensionskammer 42. Sobald das Ventil 23 wieder geöffnet wird, entspannt sich das komprimierte Medium beziehungsweise die Feder oder das elastische Element und der Kolben 30 wird entgegen der Kompressionsrichtung nach außen gedrückt, wodurch eine entsprechende Bewegung in der orthetischen oder prothetischen Vorrichtung bewirkt oder unterstützt wird.

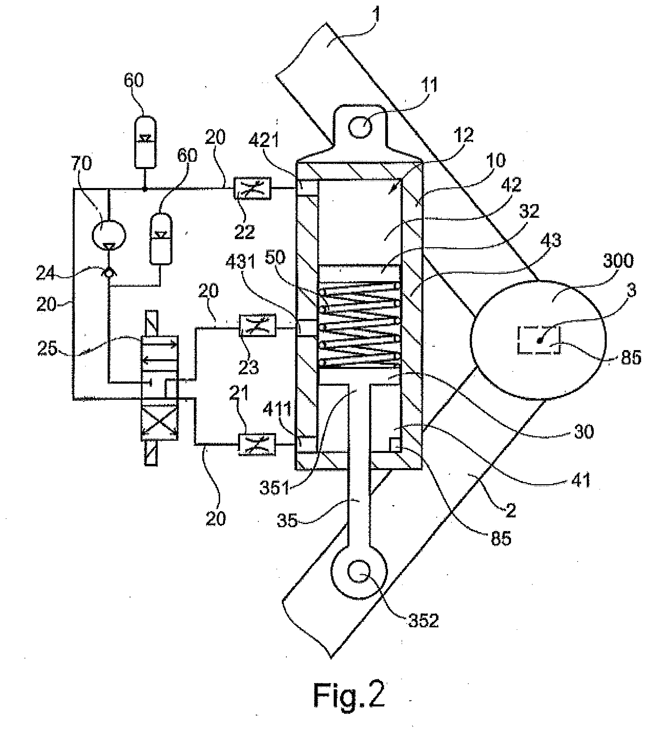

Eine Variante der Erfindung ist in der Figur 2 dargestellt, bei der die AD-Einheit mit dem Gehäuse 10 an einem Oberteil 1 einer orthetischen oder prothetischen Gelenkeinrichtung über ein Befestigungselement 11, beispielsweise einen Bolzen, befestigt ist. An dem Oberteil 1 ist ein Unterteil 2 drehbar um eine Schwenkachse 3 gelagert, die in einer Gelenkeinrichtung 300 ausgebildet ist. Die Gelenkeinrichtung verbindet das Oberteil 1 mit dem Unterteil 2 und kann als eine einfache Schwenkachse 3 oder als eine Polyzentrik ausgebildet sein. Die Kolbenstange 35 des Kolbens 30 ist mit dem zweiten Ende 352 schwenkbar an dem Unterteil 2 festgelegt. Wird der Kolben 30 nach unten bewegt, extendiert die Gelenkeinrichtung, wird der Kolben 30 nach oben bewegt, verringert sich der Gelenkwinkel, die Gelenkeinrichtung flektiert. Um die Gelenkachse 3 herum kann ein Sensor 85 zur Erfassung der Relativposition, z.B. eines Winkels, zwischen dem Oberteil 1 und dem Unterteil 2 angeordnet sein, über den Positionsdaten, z.B. Winkeldaten an eine Steuerungseinrichtung 80 übermittelt werden. Bei einem polyzentrischen Gelenk erfolgt ebenfalls eine sensorbasierte Winkelmessung, die jedoch auch über mehrere Sensoren 85 erfolgen kann. Die Sensoren 85 in Gestalt von Drucksensoren innerhalb der AD-Einheit sind nur angedeutet und liefern ebenfalls Steuersignale für die Steuereinheit 80, die nur angedeutet ist und einen Ventilblock ansteuert, der Teil der Steuereinheit 80 sein kann. Alternativ oder ergänzend kann auch die Position des Kolbens 30 und die von der AD-Einheit aufgebrachte Kraft gemessen und der Steuerungseinrichtung 80 übermittelt werden.

Der mechanische und hydraulische Aufbau der Ausgestaltungsform gemäß Figur 2 entspricht im Wesentlichen dem der Figur 1, auch hier sind drei Zugangsöffnungen 411, 421, 431 vorhanden, denen jeweils ein Stellventil 21, 22, 23 zugeordnet ist. Das jeweilige Stellventil 21, 22, 23 wird aufgrund von Steuersignalen der Steuereinheit 80 per Verstelleinrichtung verstellt, also der Strömungsquerschnitt der Fluidleitung 20 vergrößert, verkleinert oder der Durchfluss gesperrt. Im dargestellten Ausführungsbeispiel ist das Ausgleichsvolumen 60 mit einem Vordruck beaufschlagbar. Darüber hinaus ist zur Bereitstellung zusätzlicher Energie eine Pumpe 70 vorgesehen, die über ein Rückschlagventil 24 in Verbindung mit einem Ausgleichsvolumen 60 oder Speicher und einem 3-Wege-Ventil 25 mit dem hydraulischen System der AD-Einheit gekoppelt ist. Über die Pumpe 70 kann zusätzliche potentielle oder kinetische Energie in das System eingebracht werden, so dass, für den Fall, dass die innerhalb der weiteren, volumenveränderbaren Fluidkammer 43 gespeicherte Energie nicht ausreicht, die gewünschte Bewegung einzuleiten oder auszuführen, zusätzliche Bewegungsenergie bereitgestellt werden kann. Die Pumpe 70 kann zusätzlich zu der gespeicherten mechanischen Energie eingesetzt werden. Ebenso ist es möglich, über die Pumpe 70 Energie in das hydraulische System einzuspeisen, so dass ein Energiespeicher 50, der im dargestellten Ausführungsbeispiel als Wendelfeder ausgebildet ist, aufgeladen werden kann. Der Energiespeicher 50 ist als elastisches Element ausgebildet, dass sowohl als Druckelement als auch als Zugelement ausgeführt sein kann. Die Ausgestaltung des Energiespeichers als elastisches Element ist nicht auf die konkrete Ausführungsform beschränkt.

In der dargestellten Schaltstellung des 3-Wege-Ventils 25 ist die Pumpe 70 abgekoppelt. Wird das Ventil 25 nach unten bewegt, entsteht eine Verbindung zwischen der Pumpe 70 und der dritten, weiteren Fluidkammer 43, so dass hier ein Druckaufbau innerhalb der Kammer 43 stattfinden kann, wenn das vorgeschaltete Stellventil 23 geöffnet ist. Die beiden anderen Fluidkammern 41, 42 sind dann über die Fluidleitung 20 und die Ventile 21, 22 miteinander verbunden. Falls durch den beaufschlagten Druck über die Pumpe 70 eine Volumenvergrößerung innerhalb der Kammer 43 stattfindet, wird diese durch das Ausgleichsvolumen 60 aufgefangen.

Wird das 3-Wege-Ventil nach oben verfahren, wird die erste Fluidkammer 41 über die Pumpe 70 mit hydraulischem Druck beaufschlagt, so dass das Volumen der dritten Fluidkammer 43 verringert wird, wodurch bei geöffnetem Ventil 23 die Feder 50 als Energiespeicher komprimiert wird.

Ist das dritte Ventil 23 der zwischen den Kolben 30, 32 gebildeten Fluidkammer 43 gesperrt, und sind die beiden anderen Ventile 23, 22 der endseitigen Fluidkammern 41, 42 offen, ist die AD-Einheit frei beweglich. Die Energie in dem Speicher wird dissipiert, wenn das Ventil 21 der ersten Ventilkammer gesperrt ist und die beiden anderen Ventile 22, 23 geöffnet sind. Energie wird gespeichert oder abgegeben, wenn das Ventil 22 der oberen Fluidkammer 42 geschlossen ist und die beiden anderen Ventile 21, 23 geöffnet sind. Ist das obere Ventil 22 geöffnet und das Ventil 23 der zentralen Fluidkammer 43 stärker gedrosselt als das Ventil 21 der ersten Fluidkammer 41, wird Energie abgegeben, jedoch in einer der Speicherbewegung entgegengesetzten Bewegungsrichtung.

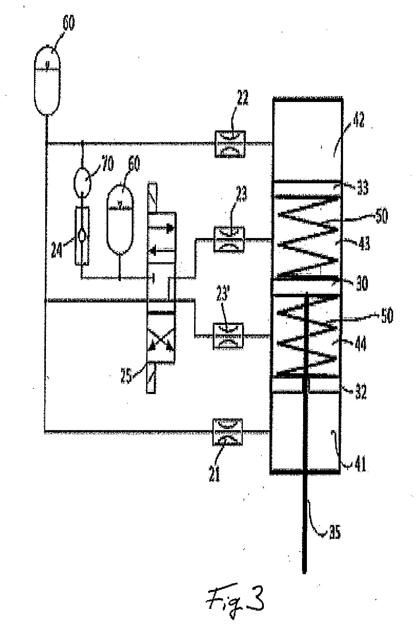

In der Figur 3 ist eine Variante der AD-Einheit gezeigt, bei der in dem Gehäuse drei Kolben 30, 32, 33 angeordnet sind, so dass insgesamt vier Fluidkammern 41, 42, 43, 44 gebildet werden. Im dargestellten Ausführungsbeispiel ist der Kolben 30, der mit der Kolbenstange 35 fest verbunden ist, zwischen den beiden weiteren Kolben 32, 33 angeordnet, wobei in den zwischen den Kolben 30, 32; 30, 33 eingeschlossenen Fluidkammern 43, 44 jeweils zumindest ein Energiespeicher 50 in Gestalt von Federn, Pneumatikkissen oder Elastomerelementen angeordnet sind. Durch das Drei-Wege-Ventil 25 lassen sich selektiv die beiden volumenvariablen, zwischen zwei Kolben ausgebildeten Kammern 43, 44 mit dem Druckfluid von der Pumpe 70 befüllen. Dazu muss das Drei-Wege-Ventil 25 entweder in die rechte oder linke Stellung bewegt werden. Ebenso lässt sich relativ leicht Druck in den beiden äußeren Kammern 41, 42 aufbauen, um ein aktive Kraft in Flexionsrichtung und Extensionsrichtung aufzubringen. Mit einer solchen Ausgestaltung der AD-Einheit ist es möglich, Energie sowohl in der Flexionsbewegung als auch in der Extensionsbewegung wahlweise zu speichern und wieder abzugeben. Durch eine Hinzuschaltung der Pumpe 70 ist es möglich, eine Kraftverstärkung bei der Abgabe von Energie in Flexions- und Extensionsrichtung bereitzustellen. Schließlich kann ein elastisches Schwingen des mittleren Kolbens 30 um die Gleichgewichtslage der Federn 43 und 44 erfolgen, das sogenannte Bouncen.

Die AD-Einheit kann sowohl als reiner Dämpfer, als auch als reiner Aktuator, als auch als Kombination aus Dämpfer mit Aktuator eingesetzt werden. Bei einer Ausgestaltung als reiner Aktuator muss keine Dämpfung vorhanden sein, bei einer Ausgestaltung als reiner Dämpfer muss keine Aktuierung oder Energiespeicherung vorhanden sein. Grundsätzlich besteht die Möglichkeit, die AD-Einheit so auszugestalten, dass zumindest eine der drei Optionen der Verwendung verwirklicht wird.

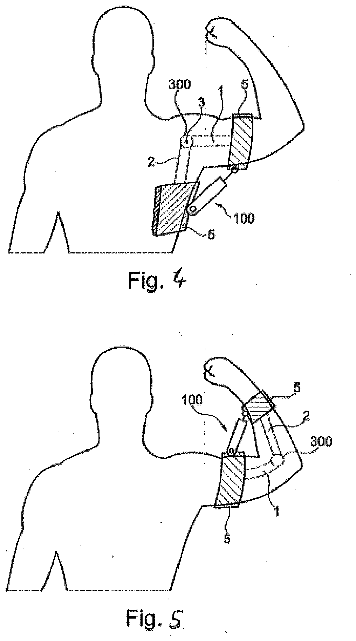

Figur 4 zeigt ein erstes Anwendungsbeispiel einer Aktuator-Dämpfereinheit 100 zur Unterstützung eines Schultergelenks. Dabei verbindet die Aktuator-Dämpfereinheit 100 ein optionales Oberteil 1 über eine Befestigungseinrichtung 5 in Gestalt einer Oberarmmanschette mit einem optionalen Unterteil 2, das ebenfalls über eine Befestigungseinrichtung 5 in Gestalt einer Thoraxschale 5 an dem Thorax eines Patienten festgelegt ist. Das Unterteil 2 ist mit dem Oberteil 1 über eine Gelenkeinrichtung 300 schwenkbar um eine Schwenkachse 3 verbunden. Das Oberteil 1 und das Unterteil 2 sind als Schiene einer Orthese ausgebildet. Beide Befestigungseinrichtungen 5 sind mit Aufnahmeeinrichtungen für die Kolbenstange 35 und das Gehäuse 10 versehen, sodass beim Einfahren oder Ausfahren der Kolbenstange 35 in das Gehäuse 10 der Arm von dem Thorax abgespreizt oder herangezogen wird. Alternativ zu einer Kopplung der Befestigungseinrichtungen 5 über einen Gelenkmechanismus 300 mit der Schwenkachse 3 und dem Oberteil 1 und dem Unterteil 2 besteht die Möglichkeit, dass der Kraftrückschluss direkt über den Körper des Patienten erfolgt, also über die Skelettstruktur, sodass das Oberteil und das Unterteil durch die Befestigungseinrichtungen 5 verwirklicht werden. Die Kraftübertragung von der Thorax-schale 5 zu dem Boden erfolgt entweder über den Körper des Anwenders oder über eine den Oberkörper und die untere Extremität umfassende Orthesenkonstruktion, ähnlich der eines Exoskelettes.

Figur 5 zeigt eine Variante in der Anwendung, bei der die Aktuator-Dämpfereinheit 100 zur Unterstützung des Ellenbogengelenkes eingesetzt wird. Dabei verwendet die Aktuator-Dämpfereinrichtung 100 eine erste Befestigungseinrichtung 5 in Gestalt einer Unterarmschale mit einer zweiten Befestigungseinrichtung 5 in Gestalt einer Oberarmschale oder einer Oberarmmanschette. Auch hier kann die orthetische oder prothetische Vorrichtung allein aus dem Aktuator-Dämpfereinrichtung 100 und den beiden Befestigungseinrichtungen 5 bestehen, optional kann der Kraftrückschluss über ein Oberteil 1 und ein Unterteil 2, die über eine Gelenkeinrichtung 300 miteinander verbunden sind, erfolgen. Das Oberteil 1 und das Unterteil 2 können jeweils als eine Schiene ausgebildet sein. Es ist auch möglich, die Ausführungsform gemäß Figur 5 mit der Ausführungsform gemäß Figur 4 zu kombinieren, sodass neben einem unterstützten Schultergelenk gemäß Figur 4 auch ein unterstütztes Ellenbogengelenk erzielt werden kann. Die weitere Krafteinleitung in den Boden erfolgt vorteilhafterweise über eine weitere, nicht dargestellte Orthesenkonstruktion oder ein Exoskelett.

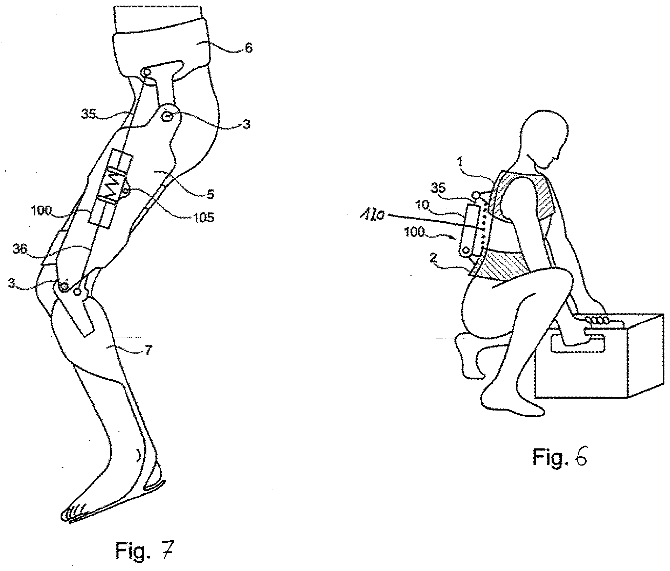

Figur 6 zeigt eine weitere Variante, bei der die Aktuator-Dämpfereinheit 100 zur Unterstützung des Rumpfes eingesetzt wird. Das Gehäuse 10 ist über eine erste Lagerstelle mit einem Oberteil 1 in Gestalt einer proximalen Körperschale und über eine zweite Lagerstelle mit einem Unterteil 2 in Gestalt einer distalen Körperschale im Bereich der Lendenwirbelsäule verbunden. Im dargestellten Ausführungsbeispiel ist die Kolbenstange 35 mit dem Oberteil 1 verbunden, grundsätzlich ist jedoch auch eine umgekehrte Zuordnung vorgesehen und möglich, sodass die Kolbenstange 35 auch an dem Unterteil 2 angeordnet sein kann. Weiterhin ist eine Kaskadierung mehrerer Aktuator-Dämpfereinheiten 100 über mehrere Segmente entlang des Rückens bzw. der Rückenwirbelsäule möglich, ggf. kann die Aktuator-Dämpfereinheit 100 auch über zwei Kolbenstangen über mehrere Gelenke wirkend ausgebildet sein. Eine mechanische Kopplung 120 zwischen dem Oberteil 1 und dem Unterteil 2 zur Realisierung eines Widerlagers für die Aktuatorkraft ist möglich und in der Figur durch eine strichlierte Linie dargestellt.

Figur 7 zeigt eine orthetische Einrichtung in Gestalt einer HKAFO (hip-knee-anklefoot-orthoses), bei der die Aktuator-Dämpfereinheit 100 an einer Oberschenkelschale 5 befestigt ist. Die Aktuator-Dämpfereinheit 100 weist zwei separate Kolben 30, 32 auf die über eine Feder oder ein Elastomerelement miteinander gekoppelt und jeweils mit einer Kolbenstange 35, 36 verbunden sind. Die Kolbenstangen 35, 36 greifen einmal an einer Hüftschale 6 und an einer Unterschenkelschale 7 an. Die Hüftschale 6 ist über ein Gelenk um eine Gelenkachse 3 schwenkbar an der Oberschenkelschale 5 gelagert. Die Drehachse 3 liegt auf der Höhe der natürlichen Hüftgelenkachse, eine Verschwenkbewegung erfolgt durch Einfahren oder Ausfahren der ersten Kolbenstange 35 in das oder aus dem Gehäuse 10. Die zweite Kolbenstange 36 ist an der Unterschenkelschiene 7 mit einem optionalen, angeformten Fußteil gelagert. Die Unterschenkelschiene 7 ist über ein Gelenk um eine Schwenkachse 3 schwenkbar relativ zu der Oberschenkelschale 5 gelagert. Auch diese Schwenkachse 3 liegt auf der Höhe der natürlichen Gelenkachse die orthetische Einrichtung übergreift mehrere Gelenke, vorliegend das Kniegelenk und das Hüftgelenk, wobei die Aktuator-Dämpfereinheit 100 über einen Anbindungspunkt 105 drehbar an der Oberschenkelschale 5 gelagert ist. Je nach Betätigung des Aktuators werden die unterschiedlichen Einfahr- und Ausfahrbewegungen der Kolbenstangen 35, 36 bewirkt, was zu einer Verschwenkung der jeweiligen Komponenten 5, 6, 7 zueinander führt. Ebenso kann durch entsprechende Schaltung von Ventilen oder Aktivierung von Magnetfeldern beim Einsatz magnetorheologischer Fluide eine Bewegungsdämpfung erreicht werden. Das Prinzip einer Orthese oder Prothese mit einem Aktuator, der mehrere Gelenke übergreift, lässt sich auch für andere Körpersegmente anwenden, insbesondere für das Knie- und Knöchelgelenk, für Rumpfsegmente oder auch für orthetische oder prothetische Einrichtungen an der oberen Extremität.

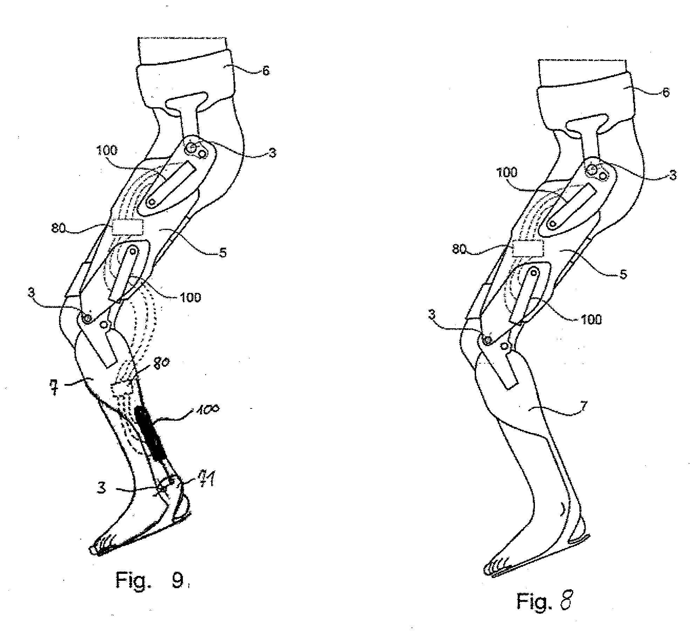

Eine Variante der Ausgestaltung einer gelenkübergreifenden orthetischen Einrichtung ist in der Figur 8 dargestellt, bei der statt eines einzelnen Aktuators bzw. einer einzelnen Aktuator-Dämpfereinheit 100 zwei Aktuator-Dämpfereinheiten 100 an einer Oberschenkelschale 5 festgelegt sind. Die einzelnen Aktuator-Dämpfereinheiten 100 sind ggf. hydraulisch miteinander gekoppelt, wobei bei der dargestellten hüftübergreifenden Beinorthese ein erster Aktuator 100 für die Bewegung der Oberschenkelschale 5 relativ zu der Hüftschale 6 oder Manschette 6 im Bereich des natürlichen Hüftgelenkes eingesetzt wird, während der zweite Aktuator 100 die Kniebewegung durch eine Verlagerung der Unterschenkelschiene 7 relativ zu der Oberschenkelschiene 5 um das Drehgelenk 3 bewirkt. Beide Aktuatoren 100 können über optionale hydraulische Leitungen, die gestrichelt dargestellt sind, miteinander hydraulisch verschaltet sein. Dazu kann eine Steuerungseinrichtung 80 oder ein Ventilsteuerblock in den Leitungen vorhanden sein, wobei durch den Ventilsteuerblock 80 verschiedene Verschaltungsvarianten einen Energietransfer zwischen den jeweiligen Aktuatoren 100 ermöglichen. Eine derartige Kopplung ist auch zwischen weiteren Aktuatoren an anderen Stellen, bzw. am Knie oder am Fuß oder zwischen beiden Beinen möglich. Die Steuerung 80 kann computergestützt arbeiten und Sensorsignale verarbeiten.

Figur 9 zeigt eine Variante der Figur 8 mit einem beweglich um eine Schwenkachse 3 an der Unterschenkelschiene 7 gelagerten Fußteil 71, das über eine AD-Einheit 100 in Richtung einer Plantarflexion und einer Dorsalflexion bewegt werden kann. Die an der Unterschenkelschale 7 gelagerte AD-Einheit 100 ist über eine separate, ggf. mit der an Oberschenkelschale 5 gelagerten Steuerung 80 gekoppelten Steuerung 80 gesteuert. Die Hydraulikströme können zwischen den AD-Einheiten 100 gekoppelt sein, um eine Kraftübertragung über die einzelne HD-Einheit 100 hinaus zu ermöglichen, so dass ggf. weniger Pumpen oder nur ein Pumpe eingesetzt werden können. Mit der Variante der Figur 9 wird das Fußteil aktuiert oder in seiner Verschwenkung relativ zu der Unterschenkelschale 7 gedämpft oder blockiert.

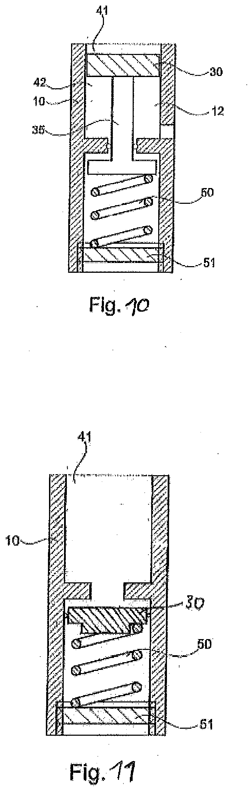

Figur 10 zeigt eine alternative Form des Energiespeichers 50, bei dem die Feder 50 des Federspeichers außerhalb des Fluidstroms, insbesondere außerhalb eines Hydraulikfluidstroms liegt. Dazu ist die Kolbenstange 35 gegenüber dem Energiespeicher 50 in Gestalt der Feder über einen Dichtring abgedichtet, so dass die Feder 50 nicht in Kontakt mit dem Hydraulikfluid, insbesondere Hydrauliköl gelangt. Dies hat den Vorteil, dass der Energiespeicher 50 auch aus einem Material bestehen kann, das mit einem Hydraulikfluid unverträglich ist. Darüber hinaus kann der Energiespeicher 50 einfach gewechselt werden, ohne dass ein Ölkreislauf geöffnet werden muss. Dazu wird lediglich eine Einstell- und Rückhaltescheibe 51 aus dem Gehäuse 10 entfernt, so dass die Feder 50 oder der Energiespeicher 50 einfach entnommen werden kann, Über die Scheibe 51 ist es auch möglich, die Vorspannung des Energiespeichers 50 zu verändern, beispielsweise wenn diese Scheibe 51 in das Gehäuse 10 eingeschraubt ist. Ein entsprechendes Gewinde ist in der Figur 30 angedeutet. Der Öldruck der jeweiligen Fluidkammer 42, 41 wirkt sich auf den Kolben 30 aus, der über den Stempel am Ende der Kolbenstange 35 den Energiespeicher 50 in Gestalt der Feder komprimiert. Das Öl aus der zweiten Fluidkammer 42 gelangt aus der Niederdruckkammer über eine Öffnung in den Niederdruckkreislauf, in dem sich auch ein Ausgleichsvolumen befindet. Das Ausgleichsvolumen nimmt die durch die Kolbenstange 35 verdrängte Ölmenge auf. Durch den Einsatz unterschiedlich steifer Federn oder Energiespeicher 50 kann die Speichercharakteristik beeinflusst und an die individuellen Bedürfnisse unterschiedlicher Patienten angepasst werden. Eine weitere Anpassung an die individuellen Wünsche der Patienten kann über die Einstellschraube oder Einstellscheibe 51 erfolgen.

Eine Variante der Figur 10 ist in der Figur 11 gezeigt, bei der der Kolben 30 entgegen einer Federkraft durch den Energiespeicher 50 in dem Gehäuse 10 gelagert ist. Der Kolben 30 ist dabei gegenüber der Gehäuseinnenwand abgedichtet, so dass das Fluid aus der Kammer 41 direkt über den Kolben die Feder 50 komprimiert und keine Kolbenstange 35, kein Stempel und kein Auslasskanal für aus der unteren Kammer 42 verdrängtes Fluid benötigt wird.

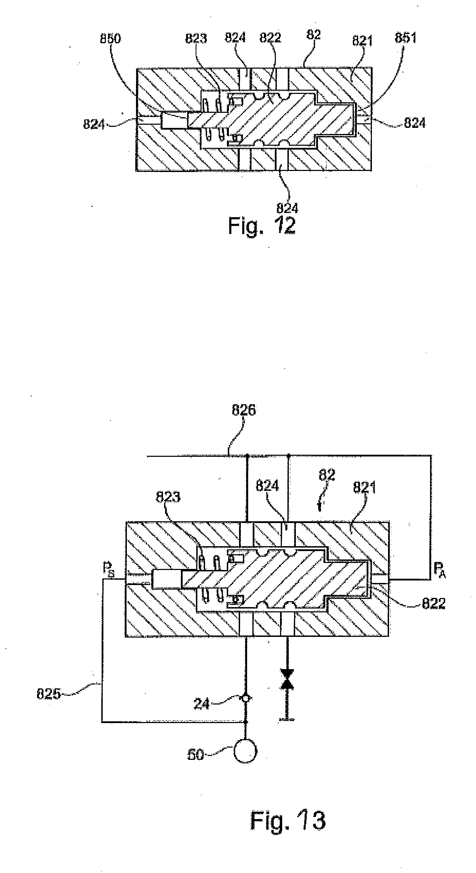

Figur 12 zeigt eine schematische Schnittdarstellung eines mechanischen Druckregelventils 82 für einen Druckspeicher mit einem Ventilkörper 821 und einem beweglich in dem Ventilkörper 821 gelagerten Schaltelement 822, das über eine Feder 823 in einer Ausgangsstellung gelagert ist. In dem Ventilkörper 821 sind mehrere Anschlussbohrungen 824 für ein Druckfluid, insbesondere ein Hydraulikfluid, angeordnet, die mit Fluidleitungen verbunden sind. Durch unterschiedliche Gestaltung der Querschnitte 850, 851 lässt sich eine Hysterese in der Schaltfunktion realisieren bzw. der Effekt der Kraft der Rückstellfeder teilweise kompensieren.

Die hydraulische Verschaltung des Druckregelventils 82 ist in der Figur 13 gezeigt. Das bewegliche Schaltelement 822 ist über eine Druckleitung 825 mit einem Fluiddruck pₛ aus dem Druckspeicher 50 beaufschlagt. Der Fluiddruck pₛ unterstützt die Druckfeder 823 und drückt das Schaltelement 822 in die dargestellte Ausgangsstellung. Wird über eine Arbeitsdruckleitung 826 Hydraulikfluid aus der Aktuator-Dämpfereinheit, die nicht dargestellt ist, dem Druckregelventil 82 zugeführt, so liegt ein Arbeitsdruck p_{A} an dem Schaltelement 822 an. Der Arbeitsdruck p_{A} wirkt gegen den Speicherdruck p_{S} und gegen die Federkraft der Feder 823. Innerhalb des beweglichen Schaltelementes 822 sind umlaufende Nuten angeordnet, die in der Ausgangsstellung einen Durchlass von der Arbeitsdruckseite zu einer Niederdruckseite ermöglichen. Ist der Arbeitsdruck p_{A} größer als der Speicherdruck ps, wird das Schaltelement 822 nach links geschoben, so dass die linke Nut innerhalb des Schaltelementes 822 zu den linken Bohrungen innerhalb des Ventilkörpers 862 ausgerichtet wird. Dadurch ergibt sich eine Strömungsverbindung von der Arbeitsdruckseite zu dem Druckspeicher 50, so dass das Arbeitsfluid nicht auf die Niederdruckseite p_{L} gelangt, sondern den Druckspeicher 50 auflädt. Ein Zurückströmen direkt durch die strömungstechnische Verbindung wird durch Rückschlagventil 24 verhindert. Wird der Speicherdruck ps zu groß, wird das Schaltelement 822 wieder nach rechts verschoben, so dass ohne Abzweigung eines Druckes für den Druckspeicher 50 das Fluid durch das Druckregelventil 82 hindurchströmen kann. Ein solches Druckregelventil 82 benötigt keinen elektronischen Steuerungsaufwand und kann ohne externe Energiezufuhr betrieben werden.

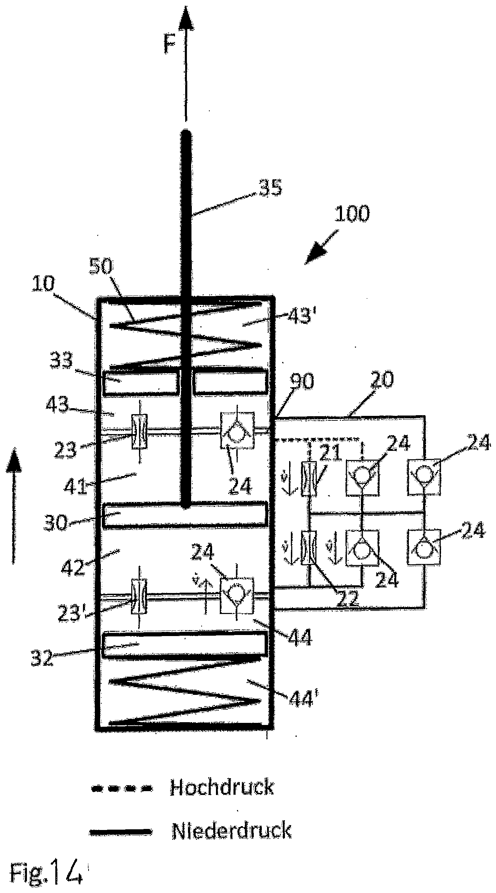

In den Figuren 14 bis 19 ist eine weitere Variante einer Aktuator-Dämpfereinheit schematisch dargestellt. Die Aktuator-Dämpfereinheit 100 als Hydraulikeinheit weist ein Gehäuse 10 mit einem darin ausgebildeten Zylinder 12 auf, in dem ein erster Kolben 30 als Arbeitskolben verschieblich gelagert ist. Der Arbeitskolben 30 ist über eine Kolbenstange 35, die aus dem Gehäuse 10 hinausführt, mit einer nicht dargestellten prothetischen oder orthetischen Einrichtung gekoppelt oder zumindest koppelbar, ebenso ist das Gehäuse an einem anderen Teil der orthetischen oder prothetischen Einrichtung festlegbar. Innerhalb des Zylinders 12 sind zwei Sperrwände 90 angeordnet, die den Zylinder 12 in insgesamt drei Kammern unterteilt, nämlich in eine mittlere Hauptkammer und zwei äußere Nebenkammern. Innerhalb dieser Kammern sind jeweils ein beweglich gelagerter Kolben 30, 32, 33 gelagert. Der mit der Kolbenstange 35 verbundene Arbeitskolben 30 ist in der mittleren Kammer angeordnet und unterteilt diese Hauptkammer in zwei Fluidkammern 41, 42. In den von den Sperrwänden 90 abgetrennten Nebenkammern sind federbelastete Kolben 32, 33 als Widerlager angeordnet, die im dargestellten Ausführungsbeispiel auf der der Hauptkammer zugewandten Seite der jeweiligen Sperrwand 90 angeordnet sind. Die Federn 50, die sich sowohl an dem jeweiligen Kolben 32, 33 als auch der Zylinderaußenwand abstützen, sind auf der dem mit der Kolbenstange 35 verbundenen Arbeitskolben 30 abgewandten Seite angeordnet. Die Federeinrichtungen 50 sind in dem dargestellten Ausführungsbeispiel als Druckfedern ausgebildet. Durch die entgegengesetzte Orientierung der Federeinrichtungen 50 kann der Arbeitskolben 30 in beiden Verlagerungsrichtungen schwingen, wobei die Federeinrichtungen 50 entsprechend der Bewegungsrichtung entspannt und gespannt werden.

Innerhalb der Sperrwände 90 sind jeweils ein Stellventil 23,23' und ein Rückschlagventil 24 angeordnet.

Die Federkolben 32, 33 oder Widerlagerkolben unterteilen die jeweilige Nebenkammer in eine mit dem Hydraulikfluid befüllbare Fluidkammer 43, 44 und eine fluidfreie Kammer 43', 44'. Innerhalb der fluidfreien Kammern 43', 44' ist der Energiespeicher oder die Federeinrichtung 50, im dargestellten Ausführungsbeispiel in Gestalt einer Feder oder eines Elastomerelementes, angeordnet. Die Kolbenstange 35 geht durch einen Kolben 33 hindurch, um aus dem Gehäuse 10 herausgeführt werden zu können.

Die durch den Arbeitskolben 30 getrennten Fluidkammern 41, 42 sind über Fluidleitungen 20, in denen zwei Stellventile 21, 22 und gegenläufig orientierte Rückschlagventile 24 angeordnet sind, hydraulisch miteinander gekoppelt. Ebenso sind die fluidgefüllten Nebenkammern 43, 44 über eine Hydraulikleitung mit gegenläufig orientierten Rückschlagventilen 24 miteinander verbunden. Die Verbindungsleitung der Nebenkammern 43, 44 ist zudem mit den Verbindungsleitungen zwischen den ersten Fluidkammern 41, 42 über eine Verbindungsleitung zwischen den beiden Rückschlagventilen 24 gekoppelt.

Die Ausführungsform stellt somit eine Kombination aus drei beweglichen Kolben 30, 32, 33 in drei Kammern und zwei Energiespeichern 50 in Gestalt von Federeinrichtungen dar. Die Energiespeicher 50 liegen außerhalb eines Fluidkontaktes an den Außenseiten der Federkolben 32, 33, während eine passive Verstellung des Arbeitskolbens 30 über die Veränderung der Bewegungswiderstände oder Antriebe innen liegt. Alternativ sind die Federeinrichtungen 50 außerhalb des Gehäuses 10 angeordnet und über beispielweise Kolbenstangen oder Hydraulikleitungen oder Pneumatikleitungen mit dem jeweiligen Widerlager 32, 33 gekoppelt. Die Durchgänge durch die Sperrwände 90 über die Stellventile 23, 23' oder die Rückschlagventile 24 können in den Sperrwänden 90 selbst angeordnet oder über außen an dem Gehäuse 10 entlanggeführte Kanäle mit entsprechenden Ventilen ausgeführt sein. Über die Stellventile 23, 23'2 und die Rückschlagventile 24 werden die Federkolben 32, 33 aus dem Kreislauf gesperrt oder eine Dämpfung bewirkt. Ebenfalls kann die passive Verstellung des Arbeitskolbens 30 durch die außerhalb des Gehäuses 10 angeordnete, parallel zu dem Arbeitskolben 30 verlaufende, zwei-Wege-Verschaltung der Hydraulikleitung gesperrt werden.

Für den Fall, dass durch die außenliegenden Stellventile 21, 22 ein hydraulischer Ausgleich gesperrt wird und nur die Durchgänge zu den Federkolben 32, 33 als Widerlager für die Federeinrichtungen 50 über die Ventile 23, 23' geöffnet sind, findet ein volumetrischer Ausgleich der Hydraulikflüssigkeit über die Federn statt. Das Volumen der fluidgefüllten Nebenkammern 43, 44 wird sich dabei je nach Stellung des Arbeitskolbens 30 ändern. Für den Fall, dass eine aktive Verstellung gesperrt ist und nur eine passive Verstellung über die außenliegenden Ventile 21, 22, 24 erfolgen soll, muss auf einen volumetrischen Ausgleich geachtet werden, hier müsste das entsprechende Stellventil 21, 22 jeweils geöffnet werden.

In der Figur 14 wird bei einer Ausfahrbewegung der Kolbenstange 35, beispielsweise bei einer Extension, der Arbeitskolben 30 in Pfeilrichtung bewegt. Dadurch wird der Druck in der oberen Fluidkammer 41 erhöht und Hochdruckfluid strömt gegen das erste Rückschlagventil 24 und durch das erste Stellventil 21, wie durch den Pfeil angedeutet, durch das zweite Stellventil 22 in die untere Fluidkammer 42. Ebenfalls gibt der untere Energiespeicher 50 Energie ab, indem er sich entspannt, wodurch Hydraulikfluid durch das untere Rückschlagventil 24 in die untere Fluidkammer 42 gefördert wird. Die Stellventile 23, 23' sind geschlossen, sodass sich eine über die Ventile 21, 22 gedämpfte Bewegung in Extensionsrichtung ergibt.

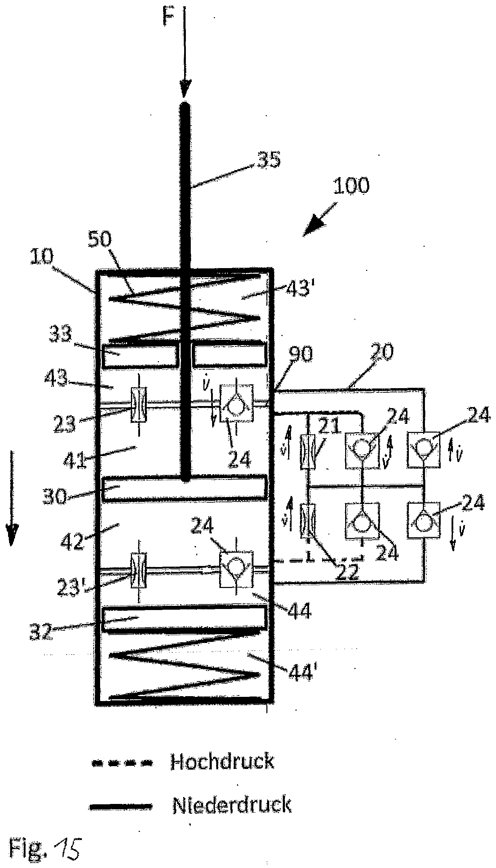

Bei einer Bewegungsumkehr, die in der Figur 15 gezeigt ist, sind die Stellventile 23, 23' in den Sperrwänden 90 weiterhin geschlossen, während die Stellventile 23, 23' in der außenliegenden Hydraulikschaltung geöffnet sind. Bei einer einfahrenden Bewegung der Kolbenstange 35, beispielsweise bei einer Flexion, strömt Hydraulikfluid von der unteren Fluidkammer 42 durch die Hochdruckleitung und das untere Stellventil 23', das obere Stellventil 23 sowie die beiden Rückschlagventile 24 sowohl in die obere Fluidkammer 41 des Arbeitskolbens 30 als auch in die obere Fluidkammer 43 des Federkolbens 31. Aufgrund des Druckes innerhalb der Verbindungsleitung zwischen dem Rückschlagventilen 24, die die Fluidkammern 43, 44 der Federkolben 32, 33 miteinander verbindet, wird gleichzeitig ein Fluidstrom in die Fluidkammer 44 des unteren Federkolbens 32 geleitet. Der obere Energiespeicher 50 entspannt sich, da er auf der Niederdruckseite angeordnet ist, sodass Hydraulikfluid aus der oberen Fluidkammer 43 durch das Rückschlagventil 24 in die obere Fluidkammer 41 des Arbeitskolbens 30 einströmen kann.

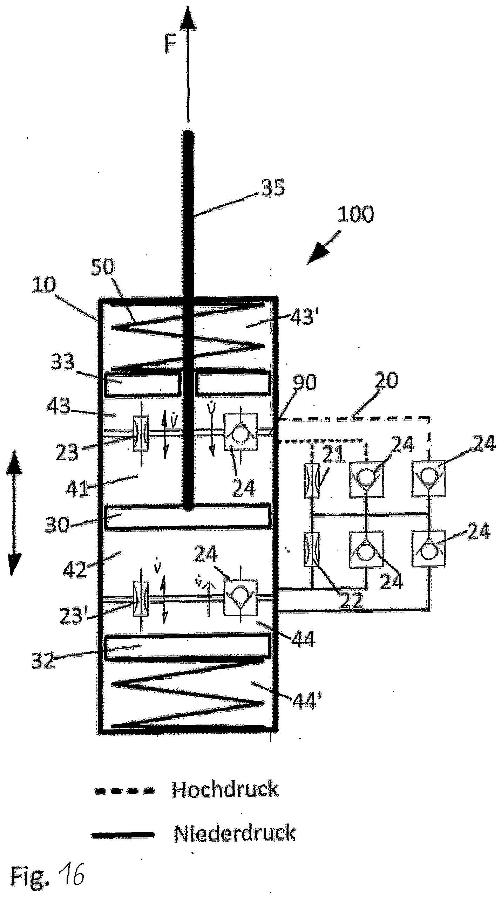

In der Figur 16 sind die Ventile 23, 23' geöffnet, während die externen Stellventile 23, 23' geschlossen sind. Durch die geöffneten Ventile 23, 23' ist eine hin- und hergehende Bewegung des Kolbens 30, wie durch den Doppelfeil angedeutet, möglich, da sich die Federn 50 in den Nebenkammern entspannen oder komprimiert werden. Der äußere Strömungsweg ist blockiert, sodass ein Volumenaustausch nur zwischen den jeweils benachbarten Fluidkammern 41, 43 und 42, 44 durch die Ventile 23, 23' und die Rückschlagventile 24 möglich ist.

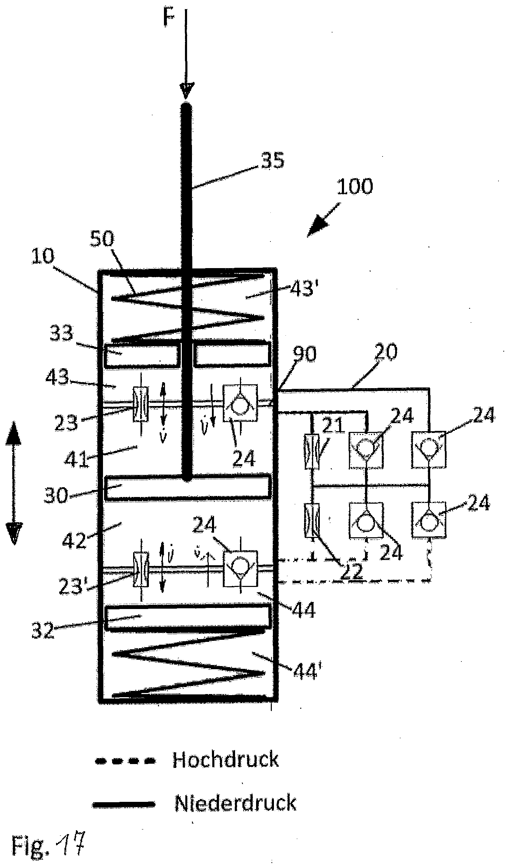

Figur 17 stellt die Situation dar, in der die Ventile analog zur Figur 16 geschaltet sind, jedoch eine Druckkraft auf die Kolbenstange 35 ausgeübt und der Arbeitskolben 30 nach unten gedrückt wird. Dementsprechend liegt die Hochdruckseite auf der Unterseite des Arbeitskolbens 30 und führt bei einer Kraftaufbringung nach unten, wie durch den Pfeil an der Kolbenstange 35 dargestellt, zu einem Einfedern der unteren Feder 50 bei einem Einfahren der Kolbenstange 35, beispielsweise bei einer Flexion und zu einem Entladen des oberen Energiespeichers 50. Entsprechend umgekehrt verhalten sich die Energiespeicher 50 bei einer umgekehrten Bewegung, also bei einem Ausfahren der Kolbenstange oder bei einer Extensionsbewegung, wie in der Figur 16 gezeigt.

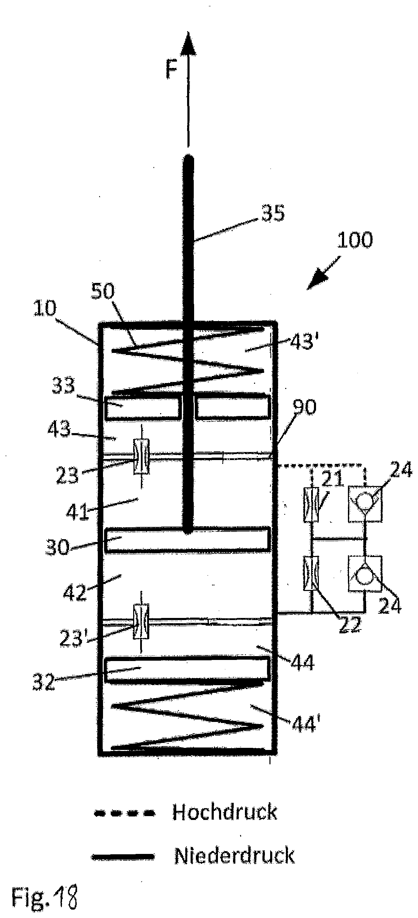

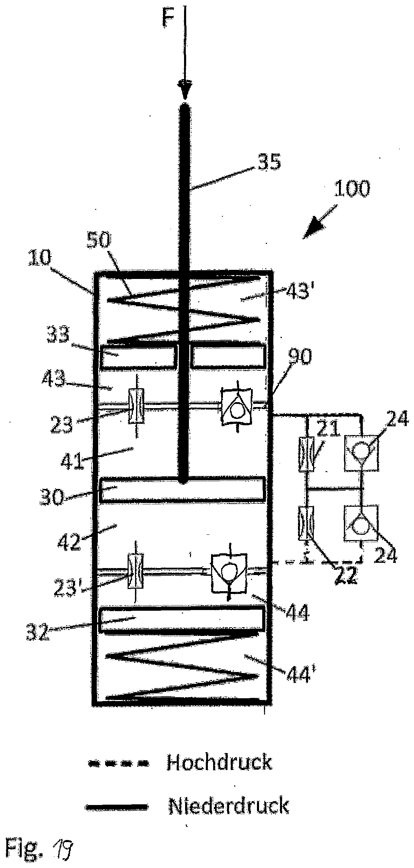

Figur 18 zeigt die Situation, bei alle Ventile 21, 22, 23, 23' geschlossen sind. Auch bei Aufbringung einer Zugkraft auf die Kolbenstange 35 ist eine Bewegung des Arbeitskolbens 30 blockiert, gleiches gilt für die umgekehrte Kraftaufbringung, die in der Figur 19 gezeigt ist.

Eine Ausgestaltung der Hydraulikeinheit 100, wie sie in den Figuren 14 bis 19 im Detail beschrieben worden ist, kann auch in den Hydraulikeinheiten, die als Aktuator-Dämpfer-Einheiten 100 bezeichnet und anhand der anderen Figuren beschrieben worden sind, eingesetzt werden.

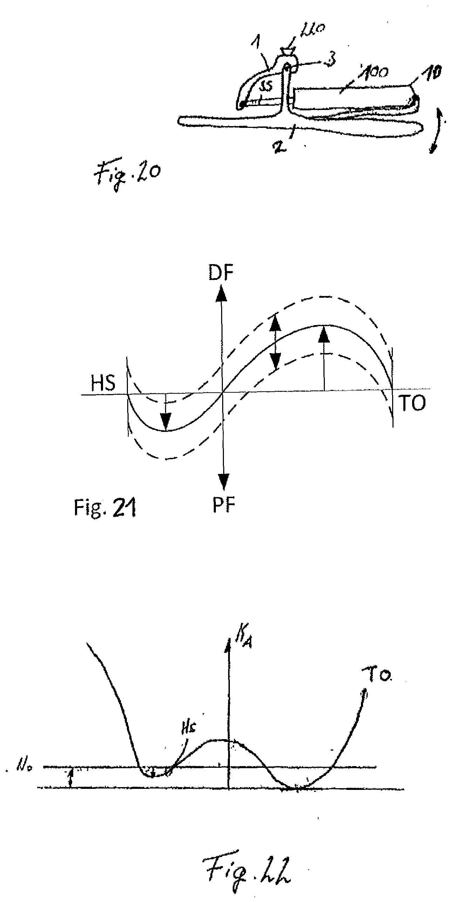

Figur 20 zeigt ein Anwendungsbeispiel der Hydraulikeinheit 100 an einem Prothesenfuß mit einem Unterteil 2 in Gestalt eines Prothesenfußunterteiles, an dem um eine Schwenkachse 3 verlagerbar ein Oberteil mit einem Pyramidenadapter 220 zur Befestigung an einem Unterschenkelrohr angeordnet ist. Das Oberteil 1 ist mit einer Kolbenstange 35 der Hydraulikeinheit 100 verbunden, die an dem gegenüberliegenden Ende der Kolbenstange 35 mit dem Gehäuse 10 an einem Ausleger des Unterteils 2 gelagert ist. Durch eine wie oben beschriebene Veränderung der Widerlager und der damit einhergehenden Verstellung der Nullpunktlage ist es möglich, eine Ausgangsstellung des Oberteils 1 relativ zu dem Unterteil 2 zu verstellen und damit den Prothesenfuß an unterschiedliche Absatzhöhen anzupassen. Die Verschwenkung und Anpassung an die Nullpunktlage ist durch den Doppelpfeil dargestellt.

In der Figur 21 ist der angenäherte Winkelverlauf zwischen dem Oberteil 1 und dem Unterteil 2 bei Anwendung in einem Prothesenfuß oder einem Orthesenfuß, beispielsweise wie in Figur 40 skizziert, dargestellt. Nach dem Aufsetzen, dem sogenannten Heel Strike HS, findet eine Plantarflexion PF statt, also eine Verlagerung der Sohle in Richtung des Bodens. Nachdem die Sohle vollständig auf dem Boden aufliegt, wird das Oberteil 1 in Gehrichtung nach vorne um die Schwenkachse 3 verlagert, der Plantarflexionswinkel nimmt wieder ab, bis sich das Unterschenkelrohr in einer senkrechten Position befindet. Anschließend wird im Verlauf eines weiteren Nachvorneverschwenkens des Unterschenkelrohrs in Gehrichtung eine Dorsalflexion DF ausgehend von der Nullpunktlage eingeleitet. Die Nullpunktlage ist bei einem Durchgang der Kurve von der Plantarflexion PF zur Dorsalflexion DF erreicht. Im weiteren Verlauf eines Schrittes kommt es zu einem sogenannten roll over und einem abnehmenden Dorsalflexionswinkel, bis die Standphase beim Gehen abgeschlossen ist, wenn bei einem toe off TO der Prothesenfuß den Boden verlässt. Die beiden oberhalb und unterhalb der durchgezogenen Linie dargestellten gestrichelten Linien zeigen die Möglichkeiten einer Verstellung der Nullpunktlage. Wird die obere Linie eingestellt, verschiebt sich die Nullpunktlage in Richtung Dorsalflexion, wird die untere Linie eingestellt, verschiebt sich die Nullpunktlage in Richtung Plantarflexion.

Figur 22 zeigt ein Beispiel eines Kniewinkelverlaufes K_{A} mit einem Nulldurchgang No, der wie durch den Doppelpfeil angedeutet, verschoben werden kann. Aus der Schwungphase kommend durchläuft der Unterschenkel einen zunehmenden Kniewinkel und komprimiert die Federeinrichtung nach Überschreiten der Linie No nach unten. Eine Extensionsbewegung erfolgt gegen eine Vorspannung durch die Feder aufgrund der Massenträgheit. Nach dem Heel Strike HS wird eine Flexion des Kniegelenkes durch Entspannung der vorgespannten Federeinrichtung unterstützt, bis zu einer maximalen Standphasenflexion, anschließend erfolgt während des roll overs eine Standphasenextension wieder gegen die Federkraft der Federeinrichtung beim Unterschreiten der Linie No. Die innerhalb der Federeinrichtung gespeicherte Energie bei Überschreiten der Nullpunktlage No wird dann am Ende der Standphase wieder abgegeben und dient zur Unterstützung der Flexionsbewegung und Einleitung der Schwungphase nach dem Fersenstoß oder toe off TO.

## Patentansprüche

1. Gelenkeinrichtung mit einem Oberteil (1) und einem Unterteil (2), die um eine Schwenkachse (3) verschwenkbar aneinander gelagert sind, mit einer an dem Oberteil (1) und dem Unterteil (2) befestigten Hydraulikeinheit (100), mit einem Gehäuse (10), in dem ein Zylinder (12) angeordnet ist, in dem ein mit einer Kolbenstange (35) verbundener Arbeitskolben (30) verlagerbar gelagert ist und den Zylinder (12) in zwei Kammern (41, 42) unterteilt, wobei das Gehäuse (10) und die Kolbenstange (35) jeweils mit dem Oberteil (1) und dem Unterteil (2) verbunden sind, **dadurch gekennzeichnet, dass** der Arbeitskolben (30) mit zumindest einer Zug- und Druckkräfte übertragenden Federeinrichtung (50) gekoppelt ist, die an zumindest einem Widerlager (32, 33) angreift, das innerhalb des Zylinders (12) angeordnet ist.

2. Gelenkeinrichtung nach Anspruch 1, **dadurch gekennzeichnet, dass** das Widerlager (32, 33) als verschieblich gelagertes Trennelement oder Kolben in der Kammer (41; 42) gelagert ist und von der Kammer (41; 42) eine Teilkammer (43, 44) abteilt.

3. Gelenkeinrichtung nach Anspruch 2, **dadurch gekennzeichnet, dass** die Kammern (41, 42) und Teilkammern (43, 44) hydraulisch miteinander über Kanäle (20) gekoppelt sind, in denen einstellbare Ventile (21, 22, 23, 24) angeordnet sind.

4. Gelenkeinrichtung nach Anspruch 1, **dadurch gekennzeichnet, dass** das Widerlager (32, 33) die Kammern (41, 42) nach außen begrenzt und eine unverschiebliche Sperrwand (90) Teilkammern (43, 44) von den Kammern (41, 42) abteilt.

5. Gelenkeinrichtung nach Anspruch 4, **dadurch gekennzeichnet, dass** jeweils die Kammern (41, 42) und die Teilkammern (43, 44) über in der Sperrwand (90) angeordnete einstellbare Ventile (23, 23') miteinander verbunden sind.

6. Gelenkeinrichtung nach Anspruch 2, **dadurch gekennzeichnet, dass** die Kammern (41, 42) hydraulisch miteinander über Kanäle (20) gekoppelt sind, in denen einstellbare Ventile (21, 22) angeordnet sind.

7. Gelenkeinrichtung nach einem der voranstehenden Ansprüche, **dadurch gekennzeichnet, dass** die Federeinrichtung (50) als Zug-Druckfeder oder als zwei gegenläufig wirkende Zug- oder Druckfedern ausgebildet sind.

8. Gelenkeinrichtung nach einem der voranstehenden Ansprüche, **dadurch gekennzeichnet, dass** die Federeinrichtung (50) innerhalb einer Kammer (41; 42) angeordnet ist.

9. Gelenkeinrichtung nach Anspruch 7, **dadurch gekennzeichnet, dass** die Zug- oder Druckfeder in unterschiedlichen Teilkammern (43, 44, 43', 44') angeordnet sind.

10. Gelenkeinrichtung nach einem der voranstehenden Ansprüche, **dadurch gekennzeichnet, dass** die Federeinrichtung (50) das Oberteil (1) relativ zu dem Unterteil (2) in einer einstellbaren Ausgangsstellung hält.

11. Gelenkeinrichtung nach einem der voranstehenden Ansprüche, **dadurch gekennzeichnet, dass** sie in einem Prothesen- oder Orthesenfuß oder einem Prothesen- oder Orthesenkniegelenk angeordnet ist.

12. Gelenkeinrichtung nach einem der voranstehenden Ansprüche, **dadurch gekennzeichnet, dass** zwei Widerlager (32, 33) in der Hydraulikeinrichtung vorhanden sind, die auf einander gegenüberliegenden Seiten des Arbeitskolbens (30) angeordnet sind.

13. Hydraulikeinheit (100) zum Einsatz in einer Gelenkeinrichtung mit einem Gehäuse (10), in dem ein Zylinder (12) angeordnet ist, in dem ein mit einer Kolbenstange (35) verbundener Arbeitskolben (30) verlagerbar gelagert ist und den Zylinder (12) in zwei Kammern (41, 42) unterteilt, **dadurch gekennzeichnet, dass** der Arbeitskolben (30) mit zumindest einer Zug- und Druckkräfte übertragenden Federeinrichtung (50) gekoppelt ist, die an zumindest einem Widerlager (32, 33) angreift, das innerhalb des Zylinders (12) angeordnet ist.

14. Verfahren zum Einstellen und Betreiben einer Gelenkeinrichtung (300) mit einen Oberteil (1) und einem Unterteil (2), die um eine Schwenkachse (3) verschwenkbar aneinander gelagert sind, mit einer an dem Oberteil (1) und dem Unterteil (2) befestigten Hydraulikeinheit (100), mit einem Gehäuse (10), in dem ein Zylinder (12) angeordnet ist, in dem ein mit einer Kolbenstange (35) verbundener Arbeitskolben (30) verlagerbar gelagert ist und den Zylinder (12) in zwei Kammern (41, 42) unterteilt, wobei das Gehäuse (10) und die Kolbenstange (35) jeweils mit dem Oberteil (1) und dem Unterteil (2) verbunden sind, **dadurch gekennzeichnet, dass** der Arbeitskolben (30) mit zumindest einer Zug- und Druckkräfte übertragenden Federeinrichtung (50) gekoppelt wird, die an zumindest einem verlagerbar in einer Kammer (41, 42) gelagerten Widerlager (32, 33) angreift, wobei die Einstellung einer Nullpunktlage des Arbeitskolbens (30) oder eine Verschwenkbewegung des Oberteils (1) relativ zu einem Unterteil (2) durch Öffnen oder Verstellen von Ventilen (21, 22) in Kanälen (20) durchgeführt wird.

15. Verfahren nach Anspruch 14, **dadurch gekennzeichnet, dass** das Widerlager (32, 33) von der Kammer (41, 42) eine Teilkammer (43, 44) abteilt und die Kammern (41, 42) und Teilkammer (43, 44) über Leitungen (20) und Ventile (21, 22, 23, 23', 24) hydraulisch miteinander gekoppelt sind und die Ventile (21, 22, 23, 23', 24) zur Sperrung der Gelenkeinrichtung (300) geschlossen werden.

16. Verfahren nach Anspruch 14, **dadurch gekennzeichnet, dass** das Widerlager (32, 33) die Kammer (41, 42) nach außen begrenzt und eine unverschiebliche Sperrwand (90) die Teilkammern (43, 44) von den Kammern (41, 42) abteilt und die Kammern (41, 42) über Leitungen (20) miteinander hydraulisch gekoppelt sind sowie jeweils die Kammer (41, 42) und die Teilkammer (43, 44) über einstellbare Ventile (23, 23') verbunden sind und die Ventile (21, 22, 23, 23') zur Sperrung der Gelenkeinrichtung (300) geschlossen werden.

17. Verfahren nach einem der Ansprüche 14 bis 16, **dadurch gekennzeichnet, dass** das Widerlager (32, 33) von der Kammer (41, 42) eine Teilkammer (43, 44) abteilt und die Kammern (41, 42) und Teilkammern (43, 44) über Leitungen (20) und Ventile (21, 22, 23, 24) hydraulisch miteinander gekoppelt sind und zur Freigabe der Gelenkeinrichtung (300) die den Kammern (41, 42) zugeordneten Ventile (21, 22) geöffnet werden.

18. Verfahren nach Anspruch 14, **dadurch gekennzeichnet, dass** das Widerlager (33, 32) die Kammer (41, 42) nach außen begrenzt und eine unverschiebliche Sperrwand (90) die Teilkammern (43, 44) von den Kammern (41, 42) abteilt und die Kammern (41, 42) über Leitungen (20) miteinander hydraulisch gekoppelt sind sowie jeweils die Kammer (41, 42) und die Teilkammer (43, 44) über in der Sperrwand (90) angeordnete einstellbare Ventile (23, 23') verbunden sind und zur Freigabe der Gelenkeinrichtung (300) die Ventile (21, 22) geöffnet werden.

19. Verfahren nach einem der Ansprüche 14 bis 18, **dadurch gekennzeichnet, dass** zur Dämpfung der Verschwenkbewegung die Ventile (21, 22, 23, 24) nur teilweise geöffnet werden.

20. Verfahren nach einem der Ansprüche 14 bis 19, **dadurch gekennzeichnet, dass** das Widerlager (32, 33) von der Kammer (41, 42) eine Teilkammer (43, 44) abteilt und die Kammern (41, 42) und die Teilkammer (43, 44) oder Teilkammern (43, 44) über Leitungen (20) und Ventile (21, 22, 23, 23') hydraulisch miteinander gekoppelt sind und zum Schwingen um einen einstellbaren Nullpunkt die hydraulische Verbindung zwischen zwei auf gegenüberliegenden Seiten des Arbeitskolbens (30) liegende Teilkammern (43, 44) geöffnet wird und die Verbindung zwischen den Kammern (41, 42) geschlossen.

21. Verfahren nach Anspruch 14, **dadurch gekennzeichnet, dass** das Widerlager (33, 32) die Kammer (41, 42) nach außen begrenzt und eine unverschiebliche Sperrwand (90) die Teilkammern (43, 44) von den Kammern (41, 42) abteilt und die Kammern (41, 42) über Leitungen (20) miteinander hydraulisch gekoppelt sind sowie jeweils die Kammer (41, 42) und die Teilkammer (43, 44) über in der Sperrwand (90) angeordnete einstellbare Ventile (23, 23') verbunden sind und zum Schwingen um einen einstellbaren Nullpunkt die hydraulische Verbindung zwischen zwei auf gegenüberliegenden Seiten des Arbeitskolbens (30) liegende Kammern (41, 42) geschlossen und die Ventile (23, 23') zwischen jeweils den gegenüber des Arbeitskolben liegenden Kammern (41, 42) und den Teilkammer (43, 44) zumindest teilweise geöffnet werden.

22. Verfahren nach einem der Ansprüche 14 bis 21, **dadurch gekennzeichnet, dass** der Gelenkeinrichtung (300) zumindest ein Sensor (85) zugeordnet ist, der mit einer Steuerungseinrichtung gekoppelt ist und einer Verstellung des Widerlagers (32, 33) auf der Grundlage von Sensorwerten erfolgt.

## Claims

1. A joint device with an upper part (1) and a lower part (2) which are mounted on each other so as to be pivotable about a pivot axis (3), with a hydraulic unit (100) secured on the upper part (1) and the lower part (2), with a housing (10) in which a cylinder (12) is arranged, in which a working piston (30) connected to a piston rod (35) is mounted displaceably and divides the cylinder (12) into two chambers (41, 42), wherein the housing (10) and the piston rod (35) are connected to the upper part (1) and the lower part (2), respectively, **characterized in that** the working piston (30) is coupled to at least one spring device (50) which transmits tensile forces and compressive forces and which engages on at least one abutment (32, 33) which is arranged inside the cylinder (12).

2. The joint device according to claim 1, **characterized in that** the abutment (32, 33) is mounted as a displaceably mounted separating element or piston in the chamber (41, 42) and separates a subchamber (43, 44) from the chamber (41, 42).

3. The joint device according to claim 2, **characterized in that** the chambers (41, 43) and subchambers (43, 44) are hydraulically connected to one another via channels (20) in which adjustable valves (21, 22, 23, 24) are arranged.

4. The joint device according to claim 1, **characterized in that** the abutment (32, 33) limits the chambers (41, 42) on the outside and a non-displaceable barrier wall (90) separates subchambers (43, 44) from the chambers (41, 42).

5. The joint device according to claim 4, **characterized in that** in each case the chambers (41, 42) and the subchambers (43, 44) are connected to one another via the adjustable valves (23, 23') arranged in the barrier wall (90).

6. The joint device according to claim 2, **characterized in that** the chambers (41, 42) are hydraulically coupled to one another via channels (20) in which adjustment valves (21, 22) are arranged.

7. The joint device according to any one of the preceding claims, **characterized in that** the spring devices (50) are configured as tensile-compressive springs or as two counteracting tensile or compressive springs.

8. The joint device according to any one of the preceding claims, **characterized in that** the spring device (50) is arranged within a chamber (41, 42).

9. The joint device according to claim 7, **characterized in that** the tensile or compressive springs are arranged in different subchambers (43, 44, 43', 44').

10. The joint device according to any one of the preceding claims, **characterized in that** the spring device (50) holds the upper part (1) in an adjustable starting position relative to the lower part (2).

11. The joint device according to any one of the preceding claims, **characterized in that** it is arranged in a prosthetic or orthotic foot or a prosthetic or orthotic knee joint.

12. The joint device according to any one of the preceding claims, **characterized in that** two abutments (32, 33) are present in the hydraulic device, which are arranged on opposite sides of the working piston (30) from one another.

13. Hydraulic unit (100) for use in a joint device with a housing (10) in which a cylinder (12) is arranged, in which a working piston (30) connected to a piston rod (35) is mounted displaceably and divides the cylinder (12) into two chambers (41, 42), **characterized in that** the working piston (30) is coupled to at least one spring device (50) which transmits tensile forces and compressive forces and which engages on at least one abutment (32, 33) which is arranged inside the cylinder (12).

14. A method for setting and operating the joint device (300) having an upper part (1) and a lower part (2), which are mounted on each other so as to be pivotable about a pivot axis (3), with a hydraulic unit (100) secured on the upper part (1) and the lower part (2), with a housing (10) in which a cylinder (12) is arranged, in which a working piston (30) connected to a piston rod (35) is mounted displaceably and divides the cylinder (12) into two chambers (41, 42), wherein the housing (10) and the piston rod (35) are connected to the upper part (1) and the lower part (2), respectively, **characterized in that** the working piston (30) is coupled to at least a spring device (50) which transmits tensile and compressive forces and which engages on at least one abutment (32, 33) mounted displaceably in a chamber (41, 42), wherein setting of a zero position of the working piston (30) or a pivoting movement of the upper part (1) relative to a lower part (2) is carried out by opening or adjusting the valves (21, 22) in the channels (20).

15. The method according to claim 14, **characterized in that** the abutment (32, 33) separates a subchamber (43, 44) from the chamber (41, 42) and the chambers (41, 42) and subchambers (43, 44) are hydraulically coupled to one another via the lines (20) and valves (21, 22, 23, 23', 24) and the valves (21, 22, 23, 23', 24) are closed to block the joint device (300).

16. The method according to claim 14, **characterized in that** the abutment (32, 33) limits the chamber (41, 42) on the outside and a non-displaceable barrier wall (90) separates the subchambers (43, 44) from the chambers (41, 42), and the chambers (41, 42) are hydraulically coupled to one another via the lines (20) and the chambers (41, 42) and the corresponding subchambers (43, 44) are connected by adjustment valves (23, 23') and the valves (21, 22, 23, 23') are closed to block the joint device (300).

17. The method according to any one of claims 14 to 16, **characterized in that** the abutment (32, 33) separates a subchamber (43, 44) from the chamber (41, 42) and the chambers (41, 42) and subchambers (43, 44) are hydraulically coupled to one another via lines (20) and valves (21, 22, 23, 24), and the values (21, 22) allocated to the chambers (41, 42) are opened to release the joint device (300).

18. The method according to claim 14, **characterized in that** the abutment (33, 32) limits the chamber (41, 42) on the outside and a non-displaceable barrier wall (90) separates the subchambers (43, 44) from the chambers (41, 42) and the chambers (41, 42) are hydraulically coupled to one another via lines (20) and each chamber (41, 42) and the corresponding subchamber (43, 44) are connected via adjustment valves (23, 23') arranged in the barrier wall (90), and the valves (21, 22) are opened to release the joint device (300).

19. The method according to any one of claims 14 to 18, **characterized in that** the valves (21, 22, 23, 24) are only partially opened for damping the pivoting movement.

20. The method according to any one of claims 14 to 19, **characterized in that** the abutment (32, 33) separates a subchamber (43, 44) from the chamber (41, 42) and the chambers (41, 42) and the subchambers (43, 44) or subchambers (43, 44) are hydraulically coupled to one another via lines (20) and valves (21, 22, 23, 23'), and for oscillation about an adjustable zero point, the hydraulic connect between two subchambers (43, 44) lying on opposite sides of the working piston (30) is opened, and the connection between the chambers (41, 42) is closed.

21. The method according to claim 14, **characterized in that** the abutment (33, 32) limits the chamber (41, 42) on the outside and a non-displaceable barrier wall (90) separates the subchambers (43, 44) from the chambers (41, 42), and the chambers (41, 42) are hydraulically coupled to one another via lines (20) and each chamber (41, 42) and the corresponding subchamber (43, 44) are connected via the adjustment valves (23, 23') arranged in the barrier wall (90), and for oscillating about an adjustable zero point, the hydraulic connection between the two chambers (41, 42) lying on opposite sides of the working piston (30) are closed, and the valves (23, 23') between the chambers (41, 42) opposite the working piston and the subchambers (43, 44) are at least partial opened.

22. The method according to any one of claims 14 to 21, **characterized in that** at least one sensor (85) is allocated to the joint device (300) and is coupled to a control device, and adjustment of the abutment (32, 33) takes place on the basis of sensor values.

## Revendications

1. Dispositif d'articulation comprenant une partie supérieure (1) et une partie inférieure (2) qui sont montées l'une contre l'autre de manière à pouvoir pivoter autour d'un axe de pivotement (3), une unité hydraulique (100) fixée à la partie supérieure (1) et à la partie inférieure (2), un boîtier (10) dans lequel est disposé un cylindre (12) dans lequel un piston de travail (30) relié à une tige de piston (35) est monté de manière à pouvoir se déplacer et subdivise le cylindre (12) en deux chambres (41, 42), le boîtier (10) et la tige de piston (35) étant reliés chacun à la partie supérieure (1) et à la partie inférieure (2), **caractérisé en ce que** le piston de travail (30) est couplé à au moins un dispositif à ressort (50) transmettant des forces de traction et de compression, qui agit sur au moins une contrebutée (32, 33) disposée à l'intérieur du cylindre (12).

2. Dispositif d'articulation selon la revendication 1, **caractérisé en ce que** la contrebutée (32, 33) est montée, en tant qu'élément de séparation ou piston monté de façon mobile en translation, dans la chambre (41 ; 42) et sépare une chambre partielle (43, 44) de la chambre (41 ; 42).

3. Dispositif d'articulation selon la revendication 2, **caractérisé en ce que** les chambres (41, 42) et les chambres partielles (43, 44) sont couplées hydrauliquement entre elles par des canaux (20) dans lesquels sont disposées des valves réglables (21, 22, 23, 24).

4. Dispositif d'articulation selon la revendication 1, **caractérisé en ce que** la contrebutée (32, 33) délimite les chambres (41, 42) vers l'extérieur, et une paroi de blocage (90) non déplaçable sépare les chambres partielles (43, 44) des chambres (41, 42).

5. Dispositif d'articulation selon la revendication 4, **caractérisé en ce que** les chambres (41, 42) et les chambres partielles (43, 44) sont chacune reliées entre elles par des valves réglables (23, 23') disposées dans la paroi de blocage (90).

6. Dispositif d'articulation selon la revendication 2, **caractérisé en ce que** les chambres (41, 42) sont couplées hydrauliquement entre elles par des canaux (20) dans lesquels sont disposées des valves réglables (21, 22).

7. Dispositif d'articulation selon l'une des revendications précédentes, **caractérisé en ce que** le dispositif à ressort (50) est réalisé sous forme de ressort de traction-compression ou sous forme de deux ressorts de traction ou de compression agissant en sens inverse.

8. Dispositif d'articulation selon l'une des revendications précédentes, **caractérisé en ce que** le dispositif à ressort (50) est disposé à l'intérieur d'une chambre (41 ; 42).

9. Dispositif d'articulation selon la revendication 7, **caractérisé en ce que** les ressorts de traction ou de compression sont disposés dans différentes chambres partielles (43, 44, 43', 44').

10. Dispositif d'articulation selon l'une des revendications précédentes, **caractérisé en ce que** le dispositif à ressort (50) maintient la partie supérieure (1) par rapport à la partie inférieure (2) dans une position initiale réglable.

11. Dispositif d'articulation selon l'une des revendications précédentes, **caractérisé en ce qu'**il est disposé dans un pied prothétique ou orthétique ou dans une articulation de genou prothétique ou orthétique.

12. Dispositif d'articulation selon l'une des revendications précédentes, **caractérisé en ce que** deux contrebutées (32, 33) sont présentes dans le dispositif hydraulique et sont disposées sur des côtés du piston de travail (30) opposés l'un à l'autre.

13. Unité hydraulique (100) destinée à être insérée dans un dispositif d'articulation comportant un boîtier (10) dans lequel est disposé un cylindre (12) dans lequel un piston de travail (30) relié à une tige de piston (35) est monté de manière à pouvoir se déplacer et subdivise le cylindre (12) en deux chambres (41, 42), **caractérisé en ce que** le piston de travail (30) est couplé à au moins un dispositif à ressort (50) transmettant des forces de traction et de compression, qui agit sur au moins une contrebutée (32, 33) disposée à l'intérieur du cylindre (12).

14. Procédé de réglage et de fonctionnement d'un dispositif d'articulation (300) comprenant une partie supérieure (1) et une partie inférieure (2) qui sont montées l'une contre l'autre de manière à pouvoir pivoter autour d'un axe de pivotement (3), une unité hydraulique (100) fixée à la partie supérieure (1) et à la partie inférieure (2), un boîtier (10) dans lequel est disposé un cylindre (12) dans lequel un piston de travail (30) relié à une tige de piston (35) est monté de manière à pouvoir se déplacer et subdivise le cylindre (12) en deux chambres (41, 42), le boîtier (10) et la tige de piston (35) étant reliés chacun à la partie supérieure (1) et à la partie inférieure (2), **caractérisé en ce que** le piston de travail (30) est couplé à au moins un dispositif à ressort (50) transmettant des forces de traction et de compression, qui agit sur au moins une contrebutée (32, 33) montée de manière déplaçable dans une chambre (41, 42), le réglage d'une position de point zéro du piston de travail (30) ou un mouvement de pivotement de la partie supérieure (1) par rapport à une partie inférieure (2) étant effectué par ouverture ou déplacement de valves (21, 22) dans des canaux (20).

15. Procédé selon la revendication 14, **caractérisé en ce que** la contrebutée (32, 33) sépare de la chambre (41, 42) une chambre partielle (43, 44), les chambres (41, 42) et la chambre partielle (43, 44) sont couplées hydrauliquement entre elles par des conduites (20) et par des valves (21, 22, 23, 23', 24), et les valves (21, 22, 23, 23', 24) sont fermées pour bloquer le dispositif d'articulation (300).

16. Procédé selon la revendication 14, **caractérisé en ce que** la contrebutée (32, 33) délimite la chambre (41, 42) vers l'extérieur, une paroi de blocage (90) non déplaçable sépare les chambres partielles (43, 44) des chambres (41, 42), les chambres (41, 42) sont couplées hydrauliquement entre elles par des conduites (20), la chambre (41, 42) et la chambre partielle (43, 44) sont chacune reliées par des valves réglables (23, 23'), et les valves (21, 22, 23, 23') sont fermées pour bloquer le dispositif d'articulation (300).

17. Procédé selon l'une des revendications 14 à 16, **caractérisé en ce que** la contrebutée (32, 33) sépare de la chambre (41, 42) une chambre partielle (43, 44), les chambres (41, 42) et les chambres partielles (43, 44) sont couplées hydrauliquement entre elles par des conduites (20) et par des valves (21, 22, 23, 24), et les valves (21, 22) associées aux chambres (41, 42) sont ouvertes pour libérer le dispositif d'articulation (300).

18. Procédé selon la revendication 14, **caractérisé en ce que** la contrebutée (33, 32) délimite la chambre (41, 42) vers l'extérieur, une paroi de blocage (90) non déplaçable sépare les chambres partielles (43, 44) des chambres (41, 42), les chambres (41, 42) sont couplées hydrauliquement entre elles par des conduites (20), la chambre (41, 42) et la chambre partielle (43, 44) sont chacune reliées par des valves réglables (23, 23') disposées dans la paroi de blocage (90), et les valves (21, 22) sont ouvertes pour libérer le dispositif d'articulation (300).

19. Procédé selon l'une des revendications 14 à 18, **caractérisé en ce que** pour amortir le mouvement de pivotement, les valves (21, 22, 23, 24) ne sont que partiellement ouvertes.

20. Procédé selon l'une des revendications 14 à 19, **caractérisé en ce que** la contrebutée (32, 33) sépare de la chambre (41, 42) une chambre partielle (43, 44), les chambres (41, 42) et la chambre partielle (43, 44) ou les chambres partielles (43, 44) sont couplées hydrauliquement entre elles par des conduites (20) et par des valves (21, 22, 23, 23') et, pour l'oscillation autour d'un point zéro réglable, la liaison hydraulique entre deux chambres partielles (43, 44) situées sur des côtés opposés du piston de travail (30) est ouverte et la liaison entre les chambres (41, 42) est fermée.

21. Procédé selon la revendication 14, **caractérisé en ce que** la contrebutée (33, 32) délimite la chambre (41, 42) vers l'extérieur, une paroi de blocage (90) non déplaçable sépare les chambres partielles (43, 44) des chambres (41, 42), les chambres (41, 42) sont couplées hydrauliquement entre elles par des conduites (20), la chambre (41, 42) et la chambre partielle (43, 44) sont chacune reliées par des valves réglables (23, 23') disposées dans la paroi de blocage (90), et, pour l'oscillation autour d'un point zéro réglable, la liaison hydraulique entre deux chambres (41, 42) situées sur des côtés opposés du piston de travail (30) est fermée, et les valves (23, 23') entre les chambres (41, 42), situées chacune à l'opposé du piston de travail, et les chambres partielles (43, 44) sont ouvertes au moins partiellement.

22. Procédé selon l'une des revendications 14 à 21, **caractérisé en ce qu'**au moins un capteur (85) est associé au dispositif d'articulation (300), lequel est couplé à un dispositif de commande, et un réglage de la contrebutée (32, 33) est effectué sur la base des valeurs du capteur.
